# EUROPEAN PATENT SPECIFICATION

(11) **EP 3 177 657 B1**
(45) Date of publication and mention of the grant of the patent: **10.07.2019**
(21) Application number: 15733151.3
(22) Date of filing: 21.04.2015
(51) Int. Cl.: C08G 18/10, A61K 8/87, A61Q 3/02

(54) **POLYURETHANE UREA SOLUTIONS FOR NAIL VARNISH COMPOSITIONS**
POLYURETHANHARNSTOFFLÖSUNGEN FÜR NAGELLACKZUSAMMENSETZUNGEN
SOLUTIONS DE POLYURÉTHANE-URÉE DESTINÉES À DES COMPOSITIONS DE VERNIS À ONGLES

(30) Priority: 05.08.2014 EP 14179782
(43) Date of publication of application: 14.06.2017
(73) Proprietor: Covestro Deutschland AG, 51373 Leverkusen (DE)
(72) Inventor: VIALA, Sophie, 50935 Koeln (DE); DOERR, Sebastian, 40593 Düsseldorf (DE); RODRIGUES, Paula Cristina Alves, 40627 Düsseldorf (DE); XIONG, Xiaohui, Shanghai 201206 (CN)
(74) Representative: Levpat
(86) International application number: PCT/CN2015/077058
(87) International publication number: WO 2015/101364

(56) References cited:
- WO-A1-99/55290
- WO-A1-2014/095164
- WO-A2-99/56705
- WO-A2-2012/061265
- CN-A- 1 524 099
- CN-A- 101 374 875
- CN-A- 101 437 484
- DE-A1- 4 241 118
- JP-A- H06 206 960
- US-A1- 2009 253 858

## Description

The present invention relates to a process for producing a nail varnish composition using polyurethane ureas, and to a nail varnish composition obtainable by the process according to the invention and to a process for producing a cosmetic coating on nails using the nail varnish compositions according to the invention.

Cosmetic formulations must have a few properties to be suitable as nail varnishes. It is particularly important here that the skin and nails are not irritated, the formulation can be applied easily, homogeneous and shiny films form on application, and the latter dry rapidly. Moreover, the film must have maximum flexibility, and have good impact resistance and good wear resistance in order to prevent cracking and flaking of the nail varnish. For this purpose, it is necessary that the cosmetic formulation is capable of forming a hard but also flexible film. Also advantageous is good stability of the hardened film to water, in order to prevent detachment from the nail on contact with water, for example on handwashing or when washing dishes.

In addition, it is often desirable that the films at first have good adhesion to the nails, but can also be removed from the nails in one piece by pulling them off. It is thus possible, for example, to avoid the use of nail varnish removers. Additionally alcohol based nail varnish removers are becoming more and more popular as they lead to less drying of nails, cuticles and skin compared to the more common nail varnish removers based on acetone or ethyl acetate. It would be desirable to have films which could be removed without any nail polish or with the aid of the more sensitive alcohol based nail varnish removers.

Film formers in nail varnish compositions have a great influence on the properties of the resulting films. The prior art, for example patent applications EP 0391322 A1, EP 0418469 A1 and EP 0423471 A2, describes the use of aqueous polyurethane dispersions in water-based nail varnishes. WO2007/115675 A1 likewise discloses aqueous nail varnishes comprising nitrocellulose-containing, aqueous polyurethane dispersions. WO 2012/061265 A1 too is concerned with nail varnishes which are produced using aqueous polyurethane dispersions inter alia. However, the aqueous polyurethane dispersions are usable only in combination with polyalkyleneamines.

However, the aqueous polyurethane dispersions used in the prior art generally have only limited compatibility with purely solvent-based nail varnish compositions.

It is also common knowledge that polyurethane ureas, because of their structure, tend to precipitate or crystallize out of organic solutions. It is therefore problematic to produce organic solutions of polyurethane ureas having a sufficiently high molecular weight without precipitation of the polyurethane ureas out of the solvents, and therefore no clear, storage-stable solutions are obtained.

For prevention of this crystallization, solvent mixtures comprising solvents which are now counted among the potentially harmful solvents on the basis of growing toxicological knowledge are recommended, for example toluene or xylene.

However, the use of such co-solvents for polyurethane ureas for use in cosmetic products is not possible in some countries for approval-related legal reasons.

WO 99/056706, WO 99/055288, WO 99/055289, WO 99/055292 and WO 99/055290 describe the use of polyurethane-based film formers for aqueous and solvent-based nail varnishes. However, ever present in these nail varnishes is a proportion of water or else likewise of cellulose-based film formers such as nitrocellulose as primary film formers. But nitrocellulose is often classified as disadvantageous in terms of compatibility for the end user. Moreover, no polyurethane urea-based systems are described therein.

There was therefore a need for film formers which are both usable as secondary film formers in purely solvent-based nail varnish compositions and can also replace cellulose-based film formers such as nitrocellulose as primary film formers.

In addition, the film formers should have the advantageous properties of polyurethane urea-based film formers, for example a high water resistance. At the same time, it would be desirable for the films obtained to be detachable from the nails without the use of nail varnish removers or with the aid of alcohol based nail varnish removers.

The problem addressed by the present invention was therefore that of providing a process for producing nail varnish compositions, and nail varnish compositions having these properties.

This problem was solved in accordance with the invention by a process for producing a nail varnish composition, characterized in that at least one polyurethane urea which has no ionically hydrophilizing groups and has been dissolved in a solvent or solvent mixture is used, the solvent consisting exclusively of one or more monohydroxy-functional alcohols or being a solvent mixture consisting of exclusively organic solvents and containing ≥ 50% by weight, based on the total mass of the solvent mixture, of at least one monohydroxy-functional alcohol,
where the polyurethane urea has been formed from
a) at least one aliphatic, araliphatic and/or cycloaliphatic diisocyanate,
b) at least one polyether polyol having a number-average molecular weight Mn of ≥ 400 and ≤ 6000 g/mol and a hydroxyl functionality of ≥ 1.5 and ≤ 4,
c) at least one amino-functional compound having at least two isocyanate-reactive amino groups,
d) optionally at least one alcohol having at least two hydroxyl groups and a molar mass of ≥ 60 and ≤ 399 g/mol,
e) optionally at least one compound having a group reactive toward isocyanate groups and
f) optionally ≤ 20% by weight, based on the total mass of the polyurethane urea, of at least one different polyol from b) having a number-average molecular weight Mn of ≥ 500 and ≤ 6000 g/mol and a hydroxyl functionality of ≥ 1.5 and ≤ 4.

The dissolved polyurethane urea used in accordance with the invention, including the solvent or solvent mixture, is also referred to hereinafter as polyurethane urea solution.

It has been found that, surprisingly, it is possible by the process according to the invention to obtain nail varnish compositions which need not contain nitrocellulose as primary film former and which also have the advantageous properties of polyurethane urea-based film formers, for example high water resistance. At the same time, the films obtained showed a sufficient adhesion to the nails, but were detachable from the nails without the use of nail varnish removers or with the aid of alcohol-based nail varnish removers.

The invention further provides for the use of a polyurethane urea which has no ionically hydrophilizing groups and has been dissolved in a solvent or solvent mixture, the solvent consisting exclusively of one or more monohydroxy-functional alcohols or being a solvent mixture consisting of exclusively organic solvents and containing ≥ 50% by weight, based on the total mass of the solvent mixture, of at least one monohydroxy-functional alcohol, for production of a nail varnish composition, where the polyurethane urea has been formed from
a) at least one aliphatic, araliphatic and/or cycloaliphatic diisocyanate,
b) at least one polyether polyol having a number-average molecular weight Mn of ≥ 400 and ≤ 6000 g/mol and a hydroxyl functionality of ≥ 1.5 and ≤ 4,
c) at least one amino-functional compound having at least two isocyanate-reactive amino groups,
d) optionally at least one alcohol having at least two hydroxyl groups and a molar mass of ≥ 60 and ≤ 399 g/mol,
e) optionally at least one compound having a group reactive toward isocyanate groups and
f) optionally ≤ 20% by weight, based on the total mass of the polyurethane urea, of at least one different polyol from b) having a number-average molecular weight Mn of ≥ 500 and ≤ 6000 g/mol and a hydroxyl functionality of ≥ 1.5 and ≤ 4.

The invention also provides for the use of a polyurethane urea which has no ionically hydrophilizing groups and has been dissolved in a solvent or solvent mixture, the solvent consisting exclusively of one or more monohydroxy-functional alcohols or being a solvent mixture consisting of exclusively organic solvents and containing ≥ 50% by weight, based on the total mass of the solvent mixture, of at least one monohydroxy-functional alcohol, for cosmetic coating of nails, where the polyurethane urea has been formed from
a) at least one aliphatic, araliphatic and/or cycloaliphatic diisocyanate,
b) at least one polyether polyol having a number-average molecular weight Mn of ≥ 400 and ≤ 6000 g/mol and a hydroxyl functionality of ≥ 1.5 and ≤ 4,
c) at least one amino-functional compound having at least two isocyanate-reactive amino groups,
d) optionally at least one alcohol having at least two hydroxyl groups and a molar mass of ≥ 60 and ≤ 399 g/mol,
e) optionally at least one compound having a group reactive toward isocyanate groups and
f) optionally ≤ 20% by weight, based on the total mass of the polyurethane urea, of at least one different polyol from b) having a number-average molecular weight Mn of ≥ 500 and ≤ 6000 g/mol and a hydroxyl functionality of ≥ 1.5 and ≤ 4.

It has been found that the polyurethane urea solutions used in accordance with the invention, in purely solvent-based nail varnish compositions, were usable advantageously both as secondary film formers in combination with cellulose-based film formers and as primary film formers in nitrocellulose-free systems.

The invention likewise provides a nail varnish composition comprising at least one plasticizer and/or special-effect constituent and at least one polyurethane urea which has no ionically hydrophilizing groups and has been dissolved in a solvent or solvent mixture, the solvent consisting exclusively of one or more monohydroxy-functional alcohols or being a solvent mixture consisting of exclusively organic solvents and containing ≥ 50% by weight, based on the total mass of the solvent mixture, of at least one monohydroxy-functional alcohol, where the polyurethane urea has been formed from
a) at least one aliphatic, araliphatic and/or cycloaliphatic diisocyanate,
b) at least one polyether polyol having a number-average molecular weight Mn of ≥ 400 and ≤ 6000 g/mol and a hydroxyl functionality of ≥ 1.5 and ≤ 4,
c) at least one amino-functional compound having at least two isocyanate-reactive amino groups,
d) optionally at least one alcohol having at least two hydroxyl groups and a molar mass of ≥ 60 and ≤ 399 g/mol,
e) optionally at least one compound having a group reactive toward isocyanate groups and
f) optionally ≤ 20% by weight, based on the total mass of the polyurethane urea, of at least one different polyol from b) having a number-average molecular weight Mn of ≥ 500 and ≤ 6000 g/mol and a hydroxyl functionality of ≥ 1.5 and ≤ 4.

The invention further provides a process for producing a cosmetic coating on nails using the nail varnish compositions according to the invention, wherein the nail varnish composition is applied to nails.

Advantageously, in the process according to the invention, the nail varnish compositions according to the invention remain at least partly on the nails.

In the context of the invention, dissolved means that, at 23°C, they are homogeneous, single-phase liquid mixtures of at least two substances which are clear. In the context of the present invention, clear means the turbidity values of the solution are ≤ 200 NTU (Nephelometric Turbidity Unit), preferably ≤ 50 NTU, particularly preferably ≤ 10 NTU and very particularly preferably ≤ 3 NTU. The turbidity values here are determined by means of a scattered-light measurement in the 90° angle (nephelometry) at a wavelength of the measurement radiation 860 nm in accordance with DIN EN ISO 7027, carried out at 23 °C using a laboratory turbidity measuring device model 2100AN from HACH LANGE GmbH, Berlin, Germany.

In the context of the invention, polyurethane ureas are polymeric compounds which have at least two, preferably at least three, urethane-group-containing repeat units 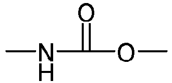 and moreover also urea-group-containing repeat units:

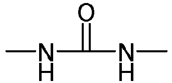

In the context of the invention, ionically hydrophilizing groups are those which could be introduced into the polyurethane urea for example by means of suitable anionically or potentially anionically hydrophilizing compounds which have at least one isocyanate-reactive group such as a hydroxyl group or amino group, and at least one functionality such as e.g. -COO-M⁺, -SO₃-M⁺, -PO(O-M⁺)₂ where M⁺ is for example metal cation, H⁺, NH₄⁺, NHR₃⁺, where R can be in each case a Cl-C12-alkyl radical, C5-C6-cycloalkyl radical and/or a C2-C4-hydroxyalkyl radical which, upon interaction with aqueous media, enters a pH-dependent dissociation equilibrium and in this way can be negatively or neutrally charged. Suitable anionically or potentially anionically hydrophilizing compounds are mono- and dihydroxycarboxylic acids, mono- and dihydroxysulfonic acids, and also mono- and dihydroxyphosphonic acids and their salts. Examples of such anionic or potentially anionic hydrophilizing agents are dimethylolpropionic acid, dimethylolbutyric acid, hydroxypivalic acid, malic acid, citric acid, glycolic acid, lactic acid and the propoxylated adduct of 2-butenediol and NaHSO₃, as is described in DE-A 2 446 440, pages 5-9, formulae I-III.

Potentially anionically groups (and also potentially ionically groups in general), are groups, which can be transformed to anionically (ionically) groups by neutralization.

In a preferred embodiment of the process according to the invention, the polyurethane urea used has no hydrophilizing groups, that means neither ionically nor nonionically hydrophilizing groups.

In the context of the invention, nonionically hydrophilizing groups are those which could be introduced into the polyurethane urea for example by means of suitable nonionically hydrophilizing compounds, such as for example polyoxyalkylene ethers which contain at least one hydroxy or amino group. Examples are monohydroxy-functional polyalkylene oxide polyether alcohols having on statistical average 5 to 70, preferably 7 to 55, ethylene oxide units per molecule, as are accessible in a manner known per se by alkoxylation of suitable starter molecules (described e.g. in Ullmann's encyclopaedia of industrial chemistry, 4th edition, volume 19, Verlag Chemie, Weinheim pp. 31-38). These compounds are either pure polyethylene oxide ethers or mixed polyalkylene oxide ethers, in which case they can then however comprise at least 30 mol%, preferably at least 40 mol%, based on all of the alkylene oxide units present, of ethylene oxide units.

In the process according to the invention for producing the nail varnish compositions, the polyurethane ureas of the present invention are dissolved in a solvent or solvent mixture, and are thus used as polyurethane urea solutions and not as aqueous dispersion.

The polyurethane urea used according to the invention is composed of
a) at least one aliphatic, araliphatic and/or cycloaliphatic diisocyanate,
b) at least one polyetherpolyol with a number-average molecular weight Mn ≥ 400 and ≤ 6000 g/mol and a hydroxyl functionality of ≥ 1.5 and ≤ 4,
c) at least one amino functional compound which has at least two isocyanate-reactive amino groups,
d) optionally at least one alcohol which has at least two hydroxyl groups and a molar mass ≥ 60and ≤ 399 g/mol,
e) optionally at least one compound which has a group that is reactive towards isocyanate groups and
f) optionally ≤ 20% by weight, based on the total mass of the polyurethane urea, of at least one polyol with a number-average molecular weight Mn ≥ 500 and ≤ 6000 g/mol and a hydroxyl functionality of ≥ 1.5 and ≤ 4, which is different from b).

In the context of this application, the number-average molecular weight is always determined by gel permeation chromatography (GPC) in tetrahydrofuran at 23°C. The procedure here is in accordance with DIN 55672-1: "Gel permeation chromatography, Part 1 - Tetrahydrofuran as eluent" (SECurity GPC System from PSS Polymer Service, flow rate 1.0 ml/min; columns: 2×PSS SDV linear M, 8×300 mm, 5 µm; RID detector). Here, polystyrene samples of known molar mass are used for the calibration. The calculation of the number-average molecular weight is assisted by software. Baseline points and evaluation limits are stipulated according to DIN 55672 Part 1.

Furthermore, the polyurethane urea is preferably composed of ≥ 5 and ≤ 60% by weight of component a), ≥ 30 and ≤ 90% by weight of component b), ≥ 2 and ≤ 25% by weight of component c), ≥ 0 and ≤ 10% by weight of component d), ≥ 0 and ≤ 10% by weight of component e) and ≥ 0 and ≤ 20% by weight of component f), in each case based on the total mass of the polyurethane urea, where the components a) to f) add up to 100% by weight.

Furthermore, the polyurethane urea is preferably composed of ≥ 10 and ≤ 40% by weight of component a), ≥ 55 and ≤ 85% by weight of component b), ≥ 5 and ≤ 20% by weight of component c), ≥ 0 and ≤ 3% by weight of component d), ≥ 0 and ≤ 3% by weight of component e) and ≥ 0 and ≤ 1% by weight of component f), in each case based on the total mass of the polyurethane urea, where the components a) to f) add up to 100% by weight.

Compounds suitable as component a) are for example 1,4-butylene diisocyanate, 1,5-pentamethylene diisocyanate (PDI), 1,6-hexamethylene diisocyanate (HDI), isophorone diisocyanate (IPDI), 2,2,4- and/or 2,4,4-trimethylhexamethylene diisocyanate, the isomeric bis(4,4'-isocyanatocyclohexyl)methanes or mixtures thereof with any desired isomer content (H12-MDI), 1,4-cyclohexylene diisocyanate, 4-isocyanatomethyl-1,8-octane diisocyanate (nonane triisocyanate), 1,3- and/or 1,4-bis(2-isocyanatoprop-2-yl)benzene (TMXDI), 1,3 -bis(isocyanatomethyl)benzene (XDI), and alkyl 2,6-diisocyanatohexanoates (lysine diisocyanates) with C1-C8-alkyl groups.

Preferably the component a) comprises no TMXDI.

Besides the polyisocyanates specified above, modified diisocyanates or triisocyanates with isocyanurate, urethane, allophanate, biuret, iminooxadiazinedione and/or oxadiazinetrione structure can also be co-used proportionately.

Preferably, they are polyisocyanates or polyisocyanate mixtures of the aforementioned type with an average NCO functionality of ≥ 2 and ≤ 4, preferably ≥ 2 and ≤ 2.6 and particularly preferably ≥ 2 and ≤ 2.4.

Preferably, the component a) is selected from aliphatic, araliphatic and/or cycloaliphatic diisocyanates which have at least one isocyanate group which is bonded to a secondary and/or tertiary carbon atom.

Particularly preferably, the component a) is selected from IPDI and/or H12-MDI.

Furthermore, preferably, no aromatic polyisocyanates are used for producing the polyurethane urea.

Component a) is preferably used in amounts of ≥ 5 and ≤ 60% by weight, particularly preferably ≥ 10 and ≤ 40% by weight and very particularly preferably from ≥ 15 and ≤ 35% by weight, based on the total weight of the polyurethane urea.

Component b) consists of one or more polyetherpolyols with a number-average molecular weight Mn ≥ 400 and ≤ 6000 g/mol and a hydroxyl functionality of ≥ 1.5 and ≤ 4, preferably with a number-average molecular weight Mn ≥ 500 and ≤ 2500 g/mol and a hydroxyl functionality of ≥ 1.9 and ≤ 3 and particularly preferably with a number-average molecular weight Mn ≥ 1000 and ≤ 2000 g/mol and a hydroxyl functionality of ≥ 1.9 and ≤ 2.1.

Suitable polyetherpolyols of component b) are for example the poly(tetramethylene glycol) polyetherpolyols known per se in polyurethane chemistry, as are available by polymerization of tetrahydrofuran by means of cationic ring-opening.

Likewise suitable polyetherpolyols are the addition products, known per se, of styrene oxide, ethylene oxide, propylene oxide, butylene oxide and/or epichlorohydrin onto di- or polyfunctional starter molecules. Thus, in particular polyalkylene glycols, such as polyethylene, polypropylene and/or polybutylene glycols, can be used, especially with the aforementioned preferred molecular weights. The polyetherpolyols here preferably have a fraction of groups obtained from ethylene oxide of < 50% by weight, preferably < 30% by weight.

Suitable starter molecules that can be used are all compounds known according to the prior art, such as for example water, butyl diglycol, glycerol, diethylene glycol, trimethyolpropane, propylene glycol, sorbitol, ethylenediamine, triethanolamine, 1,4-butanediol.

Preferably, component b) is selected from polypropylene glycols and/or poly(tetramethylene glycol) polyetherpolyols, particularly preferably selected from poly(tetramethylene glycol) polyetherpolyols.

In a preferred embodiment of the invention, component b) is one or more poly(tetramethylene glycol) polyetherpolyols with a with a number-average molecular weight Mn ≥ 500 and ≤ 2500 g/mol and a hydroxyl functionality of ≥ 1.9 and ≤ 2.1.

In a particularly preferred embodiment, component b) is a mixture of poly(tetramethylene glycol) polyetherpolyols I with a number-average molecular weight Mₙ of ≥ 400 and ≤ 1500 g/mol, particularly preferably of ≥ 600 and ≤ 1200 g/mol, very particularly preferably of 1000 g/mol and poly(tetramethylene glycol) polyetherpolyols II with a number-average molecular weight Mₙ of ≥ 1500 and ≤ 8000 g/mol, particularly preferably of ≥ 1800 and ≤ 3000 g/mol, very particularly preferably of 2000 g/mol.

The weight ratio of the poly(tetramethylene glycol) polyetherpolyols I to the poly(tetramethylene glycol) polyetherpolyols II is preferably in the range from ≥ 0.1 and ≤ 10, particularly preferably in the range from ≥ 0.2 and ≤ 8, very particularly preferably in the range from ≥ 1 and ≤ 6.

Component b) is preferably used in amounts of ≥ 30 and ≤ 90% by weight, particularly preferably ≥ 50 and ≤ 85% by weight, very particularly preferably ≥ 55 and ≤ 75% by weight, based on the total weight of the polyurethane urea.

Component c) is one or more amino-functional compounds which have at least two isocyanate-reactive amino groups.

Of suitability as component c) are for example di- or polyamines such as 1,2-ethylenediamine, 1,2- and 1,3-diaminopropane, 1,4-diaminobutane, 1,6-diaminohexane, 2,2,4- and 2,4,4-trimethylhexamethylenediamine, 2-methylpentamethylenediamine, diethylenetriamine, triaminononane, 1,3- and 1,4-xylylenediamine, α,α,α',α'-tetramethyl-1,3- and -1,4-xylylenediamine and 4,4'-diaminodicyclohexylmethane (H12-MDA), isophoronediamine (IPDA) and/or 1,2-dimethylethylenediamine.

Component c) is preferably selected from ethylenediamine, IPDA and/or H12-MDA, particularly preferably from isophoronediamine and/or H12-MDA and component c) is very particularly preferably H12-MDA.

The compounds of component c) preferably contain no hydrophilizing groups, in particular no ionically or potentially ionically hydrophilizing groups.

In a particularly preferred embodiment of the invention, component c) is selected from amines which have at least two isocyanate-reactive amino groups which are bonded to primary and/or secondary carbon atoms.

Component c) is furthermore preferably selected from symmetrically structured diamines.

Component c) is very particularly preferably selected from symmetrical diamines which have at least two amino groups which are bonded to primary and/or secondary carbon atoms, component c) being particularly preferably H12-MDA.

Component c) is preferably used in amounts of ≥ 2 and ≤ 25% by weight, particularly preferably ≥ 5 and ≤ 20% by weight and very particularly preferably ≥ 9 and ≤ 16% by weight, based on the total weight of the polyurethane urea.

In a preferred embodiment of the invention, either component a) is H12-MDI or component c) is H12-MDA or component a) is H12-MDI and component c) is H12-MDA.

Optionally, the polyurethane urea is moreover composed of component d), one or more alcohols which have at least two hydroxyl groups and a molar mass of ≥ 60 and ≤ 399 g/mol, such as for example polyols of the stated molar mass range with up to 20 carbon atoms, such as ethylene glycol, diethylene glycol, triethylene glycol, 1,2-propanediol, 1,3-propanediol, 1,4-butanediol, 1,3-butylene glycol, cyclohexanediol, 1,4-cyclohexanedimethanol, 1,6-hexanediol, neopentyl glycol, hydroquinone dihydroxyethyl ether, bisphenol A (2,2-bis(4-hydroxyphenyl)propane), hydrogenated bisphenol A, (2,2-bis(4-hydroxycyclohexyl)propane), trimethylolpropane, glycerol, pentaerythritol.

Component d) is preferably used in amounts of ≥ 0 and ≤ 10% by weight, particularly preferably ≥ 0 and ≤ 3% by weight, based on the total weight of the polyurethane urea, and is very particularly preferably not used at all.

Furthermore, the polyurethane urea can be composed of component e), one or more compounds which have a group that is reactive towards isocyanate groups, in particular compounds which have an amino or hydroxyl group. Suitable compounds of components e) are for example methylamine, ethylamine, propylamine, butylamine, octylamine, laurylamine, stearylamine, isononyloxypropylamine, dimethylamine, diethylamine, dipropylamine, dibutylamine, N-methylaminopropylamine, diethyl(methyl)aminopropylamine, morpholine, piperidine, methanol, ethanol, isopropanol, n-propanol, n-butanol, ethylene glycol monobutyl ether, diethylene glycol monomethyl ether, diethylene glycol monobutyl ether, propylene glycol monomethyl ether, dipropylene glycol monomethyl ether, tripropylene glycol monomethyl ether, dipropylene glycol monopropyl ether, propylene glycol monobutyl ether, dipropylene glycol monobutyl ether, tripropylene glycol monobutyl ether, 2-ethylhexanol, 1-octanol, 1-dodecanol, 1-hexadecanol.

Component e) preferably comprises no monofunctional polyetherpolyols which have a fraction of groups obtained from ethylene oxide of > 30% by weight, preferably > 50% by weight.

The monohydroxy-functional alcohol used as solvent for the polyurethane urea can likewise serve as structural component e) for the polyurethane urea.

Component e) is preferably used in amounts of ≥ 0 and ≤ 10% by weight, particularly preferably ≥ 0 and ≤ 3% by weight, based on the total weight of the polyurethane urea, and is very particularly preferably not used at all, in which case the monohydroxy-functional alcohol used as solvent for the polyurethane urea is not taken into consideration here as component e).

The monohydroxy-functional alcohol which serves as solvent for the polyurethane urea constitutes preferably ≥ 0 and ≤ 5% by weight, particularly preferably ≥ 0.01 and ≤ 3% by weight and very particularly preferably ≥ 0.01 and ≤ 2% by weight, of the total mass of the polyurethane urea.

Optionally, the polyurethane urea can also be composed of the component f), one or more polyols with a number-average molecular weight Mn ≥ 500 and ≤ 6000 g/mol and a hydroxyl functionality of ≥ 1.5 and ≤ 4, where the polyols are different from b).

Component f) is preferably used in amounts of ≥ 0 and ≤ 20% by weight, particularly preferably ≥ 0 and ≤ 10% by weight, based on the total weight of the polyurethane urea, and is very particularly preferably not used at all.

Preferably, the polyols of component f) have a number-average molecular weight Mn ≥ 1000 and ≤ 3000 g/mol and a hydroxyl functionality of ≥ 1.8 and ≤ 3.

Polyols suitable as component f) are the polyesterpolyols, polyacrylatepolyols, polyurethanepolyols, polycarbonatepolyols, polyesterpolyacrylatepolyols, polyurethanepolyacrylatepolyols, polyurethanepolyesterpolyols, polyurethanepolyetherpolyols, polyurethanepolycarbonatepolyols, polyetherpolycarbonatepolyols and/or polyesterpolycarbonatepolyols, in particular polyesterpolyols and/or polycarbonatepolyols, known per se in polyurethane coating technology.

Polyesterpolyols are for example the polycondensates, known per se, of di- and optionally tri-, and tetraols and di- and optionally tri- and tetracarboxylic acids or hydroxycarboxylic acids or lactones. Instead of the free polycarboxylic acids, the corresponding polycarboxylic anhydrides or corresponding polycarboxylic acid esters of lower alcohols can also be used for preparing the polyesters.

Examples of diols suitable for this purpose are ethylene glycol, butylene glycol, diethylene glycol, triethylene glycol, polyalkylene glycols such as polyethylene glycol, also 1,2-propanediol, 1,3-propanediol, butanediol(1,3), butanediol(1,4), hexanediol(1,6) and isomers, neopentyl glycol or hydroxypivalic acid neopentyl glycol ester, with hexanediol(1,6) and isomers, neopentyl glycol and hydroxypivalic acid neopentyl glycol ester being preferred. In addition, it is also possible to use polyols such as trimethylolpropane, glycerol, erythritol, pentaerythritol, trimethylolbenzene or trishydroxyethyl isocyanurate.

Dicarboxylic acids which can be used are phthalic acid, isophthalic acid, terephthalic acid, tetrahydrophthalic acid, hexahydrophthalic acid, cyclohexanedicarboxylic acid, adipic acid, azelaic acid, sebacic acid, glutaric acid, tetrachlorophthalic acid, maleic acid, fumaric acid, itaconic acid, malonic acid, suberic acid, 2-methylsuccinic acid, 3,3-diethylglutaric acid and/or 2,2-dimethylsuccinic acid. As acid source, it may also be possible to use the corresponding anhydrides.

If the average hydroxyl functionality of the polyol to be esterified is greater than 2, then monocarboxylic acids, such as benzoic acid and hexanecarboxylic acid, can additionally also be co-used.

Preferred acids are aliphatic or aromatic acids of the aforementioned type. Particular preference is given to adipic acid, isophthalic acid and optionally trimellitic acid, very particularly preferably adipic acid.

Hydroxycarboxylic acids which can be co-used as reaction participants in the preparation of a polyesterpolyol with terminal hydroxyl groups are, for example, hydroxycaproic acid, hydroxybutyric acid, hydroxydecanoic acid, hydroxystearic acid and the like. Suitable lactones are caprolactone, butyrolactone and homologues. Preference is given to caprolactone.

In component f), it is also possible for polycarbonates having hydroxyl groups, preferably polycarbonatediols, with number-average molecular weights Mn of from 400 to 8000 g/mol, preferably from 600 to 3000 g/mol, to be used. These are obtainable by reaction of carbonic acid derivatives, such as diphenyl carbonate, dimethyl carbonate or phosgene, with polyols, preferably diols.

Examples of such diols are ethylene glycol, 1,2- and 1,3-propanediol, 1,3- and 1,4-butanediol, 1,6-hexanediol, 1,8-octanediol, neopentyl glycol, 1,4-bishydroxymethylcyclohexane, 2-methyl-1,3-propanediol, 2,2,4-trimethylpentanediol-1,3, dipropylene glycol, polypropylene glycols, dibutylene glycol, polybutylene glycols, bisphenol A and lactone-modified diols of the aforementioned type. The polycarbonates having hydroxyl groups are preferably linear in structure.

In a preferred embodiment of the invention, the polyurethane urea used according to the invention is composed of
a) at least one aliphatic, araliphatic and/or cycloaliphatic diisocyanate which has at least one isocyanate group which is bonded to a secondary and/or tertiary carbon atom,
b) at least one polyetherpolyol with a number-average molecular weight Mn ≥ 500 and ≤ 2500 g/mol and a hydroxyl functionality of ≥ 1.9 and ≤ 3,
c) at least one amino-functional compound which has at least two (isocyanate-reactive) amino groups and is selected from ethylenediamine, IPDA and/or H12-MDA,
d) optionally at least one alcohol which has at least two hydroxyl groups and a molar mass ≥ 60 and ≤ 399 g/mol,
e) optionally at least one compound which has a group that is reactive towards isocyanate groups and
f) optionally ≤ 20% by weight, based on the total mass of the polyurethane urea, of at least one polyol with a number-average molecular weight Mn ≥ 500 and ≤ 6000 g/mol and a hydroxyl functionality of ≥ 1.5 and ≤ 4, which is different from b).

Furthermore preferably, the polyurethane urea in this aforementioned embodiment is composed of ≥ 5 and ≤ 60% by weight of component a), ≥ 30 and ≤ 90% by weight of component b), ≥ 2 and ≤ 25% by weight of component c), ≥ 0 and ≤ 10% by weight of component d), ≥ 0 and ≤ 10% by weight of component e) and ≥ 0 and ≤ 20% by weight of component f), in each case based on the total mass of the polyurethane urea, where the components a) to f) add up to 100% by weight.

Particularly preferably, the polyurethane urea in this aforementioned embodiment is composed of ≥ 10 and ≤ 40% by weight of component a), ≥ 55 and ≤ 85% by weight of component b), ≥ 5 and ≤ 20% by weight of component c), ≥ 0 and ≤ 3% by weight of component d), ≥ 0 and ≤ 3% by weight of component e) and ≥ 0 and ≤ 1% by weight of component f), in each case based on the total mass of the polyurethane urea, where the components a) to f) add up to 100% by weight.

In a particularly preferred embodiment of the invention, the polyurethane urea used according to the invention is composed of
a) at least one diisocyanate selected from IPDI and/or H12-MDI,
b) at least one polyetherpolyol with a number-average molecular weight Mn ≥ 500 and ≤ 2500 g/mol and a hydroxyl functionality of ≥ 1.9 and ≤ 3, selected from polypropylene glycols and/or poly(tetramethylene glycol) polyetherpolyols,
c) at least one amino-functional compound selected from IPDA and/or H12-MDA,
d) optionally at least one alcohol which has at least two hydroxyl groups and a molar mass ≥ 60 and ≤ 399 g/mol,
e) optionally at least one compound which has a group which is reactive towards isocyanate groups and
f) optionally ≤ 20% by weight, based on the total mass of the polyurethane urea, of at least one polyol with a number-average molecular weight Mn ≥ 500 and ≤ 6000 g/mol and a hydroxyl functionality of ≥ 1.5 and ≤ 4, which is different from b).

Furthermore preferably, the polyurethane urea in this aforementioned embodiment is composed of ≥ 5 and ≤ 60% by weight of component a), ≥ 30 and ≤ 90% by weight of component b), ≥ 2 and ≤ 25% by weight of component c), ≥ 0 and ≤ 10% by weight of component d), ≥ 0 and ≤ 10% by weight of component e) and ≥ 0 and ≤ 20% by weight of component f), in each case based on the total mass of the polyurethane urea, where the components a) to f) add up to 100% by weight.

Particularly preferably, the polyurethane urea in this aforementioned embodiment is composed of ≥ 10 and ≤ 40% by weight of component a), ≥ 55 and ≤ 85% by weight of component b), ≥ 5 and ≤ 20% by weight of component c), ≥ 0 and ≤ 3% by weight of component d), ≥ 0 and ≤ 3% by weight of component e) and ≥ 0 and ≤ 1% by weight of component f), in each case based on the total mass of the polyurethane urea, where the components a) to f) add up to 100% by weight.

Preferably, the polyurethane urea is composed exclusively of the components a) to c) and optionally d) to f), particularly preferably exclusively of the components a) to c).

The polyurethane urea advantageously has a number-average molecular weight Mn ≥ 2000 and ≤ 50 000 g/mol, particularly advantageously ≥ 3000 and ≤ 30 000 g/mol.

The polyurethane urea is preferably prepared by reacting the components a) and b), and optionally d) and f) in a first step to give an NCO-terminated prepolymer, which is then reacted in a subsequent step with the component c) and optionally the components d) and e).

For the preparation of the polyurethane ureas, the components a) and b) and also optionally d) and f) for the preparation of an NCO-terminated prepolymer are completely or partially introduced, optionally diluted with a solvent that is inert towards isocyanate groups and heated to temperatures in the range from 50 to 120°C. To increase the rate of the isocyanate addition reaction, the catalysts known in polyurethane chemistry can be used. In one preferred variant, however, processing takes place without the addition of urethanization catalysts.

Then, the constituents of a) and b) and also optionally d) and f) possibly still not added at the start of the reaction are metered in.

During the preparation of the NCO-terminated prepolymers of components a) and b) and also optionally d) and f), the quantitative ratio of isocyanate groups to isocyanate-reactive groups is generally ≥ 1.05 and ≤ 3.5, preferably ≥ 1.1 and ≤ 3.0, particularly preferably ≥ 1.1 and ≤ 2.5.

Isocyanate-reactive groups are to be understood as meaning all groups that are reactive towards isocyanate groups, such as for example primary and secondary amino groups, hydroxy groups or thiol groups.

The reaction of components a) and b) and also optionally d) and f) to give the prepolymer takes place partially or completely, but preferably completely. Polyurethane prepolymers which contain free isocyanate groups are thus obtained in bulk or in solution.

Preferably, the NCO-terminated prepolymer is prepared exclusively from components a) and b).

Then, preferably in a further process step, if this has still not taken place or has taken place only partially, the resulting prepolymer is dissolved with the help of one or more organic solvents. The solvent used here is preferably likewise a solvent or solvent mixture, where the solvent consists exclusively of one or more monohydroxy-functional alcohols, or a solvent mixture consisting of exclusively organic solvents which comprises ≥ 50% by weight, based on the total mass of the solvent mixture, of at least one monohydroxy-functional alcohol, is used. For the solvent and the solvent mixture, the preferred embodiments below relating to solvent or solvent mixture in which the polyurethane urea is dissolved are likewise applicable. The solvent or solvent mixture here can also be different from the solvent or solvent mixture in which the polyurethane urea is later dissolved as end product. Preferably, the solvent or solvent mixture is identical to the solvent or solvent mixture in which the polyurethane urea is later dissolved as end product.

Preferably, the solvent used in the preparation consists of one or more monohydroxy-functional alcohols.

The ratio of solvent to prepolymer is here preferably ≥ 1:10 and ≤ 5:1, particularly preferably ≥ 1:2 and ≤ 2:1 parts by weight.

The prepolymer is preferably cooled prior to the dissolution to temperatures of -20 to 60°C, preferably 0 to 50°C and particularly preferably from 15 to 40°C.

In a further step, which optionally follows the dissolution of the NCO-terminated prepolymer, the NCO-terminated prepolymer obtained in the first step is then preferably reacted completely or partially with component c) and also optionally components d) and e). This reaction is generally referred to as chain extension, or in the case of component e) as chain termination.

Preferably here, the NCO-terminated prepolymer is initially introduced and component c) and also optionally d) and e) are metered in. Preferably, firstly a partial reaction of the NCO groups of the prepolymer with component c) and optionally d) takes place, and then the chain termination by reaction of the remaining NCO groups with component e). Components c) and optionally e) can also be added here in stages in several steps, in particular in two steps.

Component c) and also optionally d) and e) are preferably used dissolved in one or more organic solvents. The solvent used here is preferably likewise a solvent or solvent mixture, where the solvent consists exclusively of one or more monohydroxy-functional alcohols, or a solvent mixture consisting of exclusively organic solvents which comprises ≥ 50% by weight, based on the total mass of the solvent mixture, of at least one monohydroxy-functional alcohol, is used. For the solvent and the solvent mixture, the preferred embodiments below relating to the solvent or solvent mixture in which the polyurethane urea is dissolved are likewise applicable.

The solvent or solvent mixture here can also be different from the solvent or solvent mixture in which the polyurethane urea is later dissolved as end product. Preferably, the solvent or solvent mixture is identical to the solvent or solvent mixture in which the polyurethane urea is later dissolved as end product.

Preferably, the solvent for component c) used in the preparation consists of one or more monohydroxy-functional alcohols.

If solvents are used as diluents, then the diluent content in components c) and also optionally d) and e) used in the chain extension is preferably 1 to 95% by weight, particularly preferably 3 to 50% by weight, based on the total weight of component c) and also optionally d) and e) including diluents.

The addition of component c) and also optionally d) and e) takes place preferably at temperatures of -20 to 60°C, preferably 0 to 50 and particularly preferably from 15 to 40°C.

The degree of chain extension, e.g. the molar ratio of NCO-reactive groups of the components c) and also optionally d) and e) used for the chain extension and chain termination to free NCO groups of the prepolymer is generally ≥ 50 and ≤ 150%, preferably ≥ 50 and ≤ 120%, particularly preferably ≥ 60 and ≤ 100% and very particularly preferably ≥ 70 and ≤ 95%.

Preferably, the molar ratio of the isocyanate-reactive groups of component c) to the free NCO groups of the prepolymer is ≥ 50 and ≤ 120%, particularly preferably ≥ 60 and ≤ 100% and very particularly preferably ≥ 70 and ≤ 95%.

In a preferred embodiment of the invention, the free NCO groups of the prepolymer are reacted only partially with component c), the molar ratio of the isocyanate-reactive groups of component c) to the free NCO groups of the prepolymer being preferably ≥ 60 and ≤ 95%, and the remaining free NCO groups react to completion with the hydroxy groups of the solvent, giving an NCO-free polyurethane urea.

Following the preparation, the polyurethane urea, if solvents or solvent mixtures according to the invention have already been used in the preparation process, can furthermore be diluted with a solvent or solvent mixture and be thereby dissolved, where the solvent consists exclusively of one or more monohydroxy-functional alcohols, or a solvent mixture consisting of exclusively organic solvents which comprises ≥ 50% by weight, based on the total mass of the solvent mixture, of at least one monohydroxy-functional alcohol, is used.

If no solvents or solvent mixtures according to the invention have been used during the reaction, then after the preparation of the polyurethane urea, the latter is used in a solvent or solvent mixture where the solvent consists exclusively of one or more monohydroxy-functional alcohols, or a solvent mixture consisting of exclusively organic solvents which comprises ≥ 50% by weight, based on the total mass of the solvent mixture, of at least one monohydroxy-functional alcohol, is used.

The dissolution of the polyurethane urea can take place using the customary techniques for shearing, for example by stirring using standard stirrers, for example as specified in DIN 28131.

The dissolution of the polyurethane urea is preferably carried out without addition of external emulsifiers. The polyurethane urea solutions used according to the invention preferably comprise no external emulsifiers.

Suitable solvents or constituents of the solvent mixture are in principle all monohydroxy-functional, aliphatic alcohols with one to six carbon atoms, such as, for example, methanol, ethanol, n-propanol, isopropanol, n-butanol, sec-butanol and/or butyl glycol. The monohydroxy-functional alcohol is particularly preferably ethanol.

If a solvent mixture is used, then besides the monohydroxy-functional alcohols, ≤ 50% by weight, based on the total mass of the solvent mixtures, of a further organic solvent can also be used. Suitable solvents are here are for example esters, such as e.g. ethyl acetate, butyl acetate, methyoxypropyl acetate or butyrolactone, ketones, such as e.g. acetone or methyl ethyl ketone, ethers, such as e.g. tetrahydrofuran or tert-butyl methyl ether, aromatic solvents, such as e.g. xylene or solvent naphtha. In the event of using ethanol, typical denaturing agents can be present as additives in the customary additive amounts.

Preferably, the fraction of the further organic solvents is ≤ 30% by weight, particularly preferably ≤ 5% by weight and very particularly preferably ≤ 2% by weight, based on the total weight of the solvent mixture. In a very particularly preferred embodiment, no further organic solvents of any kind are present besides monohydroxy-functional, aliphatic alcohols.

Further solvents that are unsuitable are physiologically nontolerable solvents such as, for example, dimethylformamide, N-methylpyrrolidone or toluene, as are often used as co-solvents for polyurethanes or polyurethane ureas, and these should preferably not be present in cosmetic compositions.

The further solvents are also not water. The polyurethane urea solution obtained by dissolving the polyurethane urea in the solvents or solvent mixtures used according to the invention is preferably anhydrous, with the exception of the fractions of water which are present in the organic solvents used as a consequence of their production.

The water fraction of the polyurethane urea solution is preferably ≤ 10% by weight, particularly preferably ≤ 4.5% by weight and very particularly preferably ≤ 1% by weight, based on the total mass of the polyurethane urea solution.

The fraction of the polyurethane urea (as active substance) in the polyurethane urea solution used according to the invention (also referred to as solids content) is here preferably ≥ 10 and ≤ 80% by weight, particularly preferably ≥ 15 and ≤ 60% by weight and very particularly preferably ≥ 20 and ≤ 50% by weight, based on the total weight of the polyurethane urea solution.

In the context of the process according to the invention for production of the nail varnish composition, the raw materials used are preferably mixed at room temperature. The components are preferably mixed with the aid of a dissolver disc over a period of 5 to 60 min. After the mixing, the components are optionally, if required, ground with a bead mill over a period of 10 to 120 min.

The invention further provides a nail varnish composition obtainable by the process according to the invention.

The invention likewise provides a nail varnish composition comprising at least one plasticizer and/or special-effect constituent and at least one polyurethane urea which has no ionically hydrophilizing groups and has been dissolved in a solvent or solvent mixture, the solvent consisting exclusively of one or more monohydroxy-functional alcohols or being a solvent mixture consisting of exclusively organic solvents and containing ≥ 50% by weight, based on the total mass of the solvent mixture, of at least one monohydroxy-functional alcohol, where the polyurethane urea has been formed from
a) at least one aliphatic, araliphatic and/or cycloaliphatic diisocyanate,
b) at least one polyether polyol having a number-average molecular weight Mn of ≥ 400 and ≤ 6000 g/mol and a hydroxyl functionality of ≥ 1.5 and ≤ 4,
c) at least one amino-functional compound having at least two isocyanate-reactive amino groups,
d) optionally at least one alcohol having at least two hydroxyl groups and a molar mass of ≥ 60 and ≤ 399 g/mol,
e) optionally at least one compound having a group reactive toward isocyanate groups and
f) optionally ≤ 20% by weight, based on the total mass of the polyurethane urea, of at least one different polyol from b) having a number-average molecular weight Mn of ≥ 500 and ≤ 6000 g/mol and a hydroxyl functionality of ≥ 1.5 and ≤ 4.

Nail varnish compositions in the context of the invention are especially nail varnishes, which serve to decorate the nails with colour, but also compositions for care and for protection of the nails, and likewise primers for nails to which further layers of nail varnishes may be applied.

Nails in the context of this invention are especially understood to mean human fingernails and/or toenails, but also synthetic nails which have already been secured to the human body or are intended for securing to the human body. Such synthetic nails are based, for example, on materials such as synthetic polymers.

The proportion of the polyurethane urea solution used in the nail varnish composition is preferably ≥ 0.5% and ≤ 80% by weight, more preferably ≥ 1% and ≤ 60% by weight and most preferably ≥ 2% and ≤ 40% by weight, based on the total mass of the nail varnish composition.

The solids content of the polyurethane urea solution is preferably chosen such that the nail varnish compositions contain preferably ≥ 0.1% and ≤ 50% by weight, more preferably ≥ 0.2% and ≤ 40% by weight and most preferably ≥ 0.5% and ≤ 30% by weight of the polyurethane urea as active substance, based on the total mass of the nail varnish composition.

Active substance is understood to mean the polyurethane urea without solvent or solvent mixture.

Preferably, the nail varnish compositions according to the invention do not contain any polyalkyleneamines. Additionally preferably, the nail varnish compositions according to the invention do not contain any alkoxysilanes having a solubilizing group in the sense of WO2012/061265.

In one embodiment of the invention, the nail varnish compositions comprise both the polyurethane urea solutions used in accordance with the invention as secondary film formers and a different primary film former. Optionally, the nail varnish composition may also comprise further secondary film formers.

The primary film-forming polymer is advantageously selected from nitrocelluloses, cellulose acetobutyrates, cellulose acetopropionates, cellulose ethers and/or mixtures thereof. Particular preference is given to nitrocelluloses. Very particular preference is given to the nitrocelluloses commercially available as the RS grade, having a nitrogen content between 11.2% and 12.8%. Within the RS grade, there are sub-types which differ particularly in terms of their molecular weight. Additionally preferably, nitrocellulose RS ⅛ sec., nitrocellulose ¼ sec., nitrocellulose RS ½ sec., nitrocellulose RS 5-6 sec. and nitrocellulose 60-80 sec. are used in the composition according to the invention. These nitrocelluloses are known in the European nomenclature as nitrocellulose E 23 to nitrocellulose E 130.

In a preferred embodiment of the invention, the polyurethane urea solution used in accordance with the invention, however, is present in the nail varnish composition as primary film former. In this embodiment, the nail varnish composition is free of nitrocellulose and other cellulose-based film formers. In that case, the nail varnish composition preferably contains further secondary film formers which are not nitrocellulose or other cellulose-based film formers.

The proportion of the primary film former in the composition according to the invention is preferably ≥ 5% and ≤ 30% by weight, more preferably ≥ 6% and ≤ 25% by weight and most preferably ≥ 8% and ≤ 20% by weight, based on the total weight of the composition.

As well as the polyurethane urea solutions used in accordance with the invention as primary or secondary film formers, it is possible to use further secondary film formers.

In an advantageous embodiment of the invention, secondary film formers used are polymers containing ester groups, more preferably polyester polyols.

The polyester polyols are advantageously the products or mixtures thereof available from Bayer Material Science AG under the following trade names:
Desmophen® 1700, Desmophen® 1652, Desmophen® VPLS 2328, Desmophen® 850, Desmophen® 1150, Desmophen® 1155, Desmophen® 1145, Desmophen® 1800, Desmophen® 670, Desmophen® 1200, Desmophen® VPLS 2068, Desmophen® 1100, Desmophen® 2400 S, Desmophen® 2450 X, Desmophen® 800, Desmophen® VPLS 2249/1, Desmophen® 690 MPA, Desmophen® T 1665 SN/IB, Desmophen® 680 X, Desmophen® T1775 SN, Desmophen® 680 BA, Desmophen® T 2082, Desmophen® T XP 2374, Desmophen® 670 BA, Desmophen® VP LS 2388, Desmophen® 650 MPA, Desmophen®651 MPA, Desmophen® 651 MPA/X, Desmophen® 800 BA, Desmophen® 800 MPA, Desmophen® PL 800, Desmophen® PL 300 X, Desmophen® 1300 BA, Desmophen® 1300 EA, Desmophen® 1300 X, Desmophen® 1400 PR, Desmophen® PL 817, Desmophen® 1388 71 EA, Baycoll® AD 2055, Baycoll® AD 2047, Baycoll® AD 1225, Baycoll® AD 5027, Baycoll® AS 2060, Baycoll® AV 2113.

Particularly advantageously, Desmophen® 680 BA is used.

Further advantageous secondary film formers which can be used in addition to or instead of the abovementioned polyester polyols are:
- tosylamide/formaldehyde resins (for example Ketjenflex® MH and Ketjenflex® MS-80 from Akzo Nobel, Sulfonex® from Estron, Santolite MHP, Santolite MS 80 from FACONNIER or RESIMPOL 80 from PAN AMERICANA)
- tosylamide/epoxy resins (for example Lustrabrite S, Lustrabrite S-70, Nagellite 3050 from Telechemische or Polytex® Resin, Polytex® NX-55 from Estron)
- adipic acid/neopentyl glycol/phthalic anhydride copolymers (for example Uniplex 670-P from Unitex Chempol Corporation)
- phthalic anhydride/glycerol/glycidyl decanoate copolymers
- phthalic anhydride/phthalic anhydride/glycol copolymers (for example Polynex Resin, 7809 Polynex Resin from Estron)
- glycerol/phthalic acid copolymers
- styrene/acrylate/acrylonitrile copolymers
- polymers and copolymers of acrylates (for example ACRYLOID B66 from Dow)
- copolymers of acrylates and styrene
- sucrose acetate isobutyrate (for example Eastman SAIB from Eastman Chemical company)
- polyvinyl butyral resins
- alkyd resins (for example BECKOSOL ODE 230-70-E from DAINIPPON)
- polyurethane resins (for example TRIXENE PR 4127 from Baxenden Chemicals Ltd.)

The proportion of the secondary film former in the composition according to the invention is preferably ≥ 0% and ≤ 30% by weight, more preferably ≥ 0.5% and ≤ 20% by weight and most preferably ≥ 1% and ≤ 15% by weight, based on the total weight of the composition.

Advantageously, the film former(s) are selected from the group of the polyurethanes other than the polyurethane ureas used in accordance with the invention, the polyureas, silicone resins and/or polyesters, and the nonionic polymers and mixtures thereof.

Advantageous nonionic polymers which may be present in nail varnish compositions according to the invention alone or in a mixture are selected from:
- polyalkyloxazolines,
- vinyl acetate homo- or copolymers. These include, for example, copolymers of vinyl acetate and acrylic esters, copolymers of vinyl acetate and ethylene, copolymers of vinyl acetate and maleic esters,
- acrylic ester copolymers, for example the copolymers of alkyl acrylate and alkyl methacrylate, copolymers of alkyl acrylate and urethanes,
- copolymers of acrylonitrile and nonionic monomer selected from butadiene and (meth)acrylate,
- styrene homo- and copolymers. These include, for example, homopolystyrene, copolymers of styrene and alkyl (meth)acrylate, copolymers of styrene, alkyl methacrylate and alkyl acrylate, copolymers of styrene and butadiene, copolymers of styrene, butadiene and vinylpyridine,
- polyamides,
- vinyllactam homo- or copolymers, such as vinylpyrrolidone homo- or copolymers; these include, for example, polyvinylpyrrolidone, polyvinylcaprolactam, copolymers of N-vinylpyrrolidone and vinyl acetate and/or vinyl propionate in various concentration ratios, polyvinylcaprolactam, polyvinylamides and salts thereof, and also copolymers of vinylpyrrolidone and dimethylaminoethyl methacrylate, terpolymers of vinylcaprolactam, vinylpyrrolidone and dimethylaminoethyl methacrylate,
- polysiloxanes,
- homopolymers of N-vinylformamide, e.g. PVF from Akzo Nobel.
- Particularly preferred nonionic polymers are acrylic ester copolymers, homopolymers of vinylpyrrolidone and copolymers, polyvinylcaprolactam.
- Very particularly preferred nonionic polymers are homopolymers of vinylpyrrolidone, for example Luviskol® K from BASF, copolymers of vinylpyrrolidone and vinyl acetate, for example Luviskol® VA products from BASF or PVPVA® S630L from Ashland, terpolymers of vinylpyrrolidone, vinyl acetate and propionate, for example Luviskol® VAP from BASF and polyvinylcaprolactams, for example Luviskol® PLUS from BASF.

The nail varnish composition according to the invention preferably further comprises a physiologically acceptable medium. Physiologically acceptable media are understood to mean substances which are toxicologically safe and are suitable for application to human body, specifically to keratin-based parts of the body such as nails.

Preferably, the physiologically acceptable medium of the composition according to the invention comprises an organic solvent or a mixture of organic solvents, and more preferably consists thereof. The preferred organic solvents are, for example:
- ketones which are liquid at room temperature, for example methyl ethyl ketone, methyl isobutyl ketone, diisobutyl ketone, isophorone, cyclohexanone, acetone;
- alcohols which are liquid at room temperature, for example ethanol, butanol, propanol, isopropanol, diacetone alcohol, 2-butoxyethanol, cyclohexanol;
- propylene glycols which are liquid at room temperature, for example propylene glycol monomethyl ether, propylene glycol monomethyl ether acetate, dipropylene glycol mono-n-butyl ether;
- cyclic ethers, for example γ-butyrolactone;
- short-chain esters having 3 to 8 carbon atoms, for example ethyl acetate, methyl acetate, propyl acetate, isopropyl acetate, n-butyl acetate, isopentyl acetate, methoxypropyl acetate, t-butyl acetate, butyl lactate;
- ethers which are liquid at room temperature, for example diethyl ether, dimethyl ether, dichlorodiethyl ether;
- alkanes which are liquid at room temperature, for example decane, heptane, dodecane, hexane, cyclohexane;
- glycols, for example ethylene glycol, propylene glycol, pentylene glycol, glycerol;
- alkyl sulphoxides, for example dimethyl sulphoxide;
- aldehydes which are liquid at room temperature, for example benzaldehyde, acetaldehyde;
- 3-ethylethoxypropionate;
- carbonates, for example propylene carbonate, dimethyl carbonate;
- acetals, for example methylal;
and mixtures of the solvents mentioned.

Particularly advantageously, the organic solvents are selected from the group of ethyl acetate, butyl acetate, propyl acetate, isobutyl acetate, ethanol, propanol, isopropanol, butanol, methyl ethyl ketone, methyl isobutyl ketone, heptane and/or hexane and mixtures thereof.

In the case of the composition according to the invention, the proportion of solvent may be within the range of ≥ 10% and ≤ 95% by weight, preferably in the range of ≥ 15% and ≤ 80% by weight, and more preferably in the range of ≥ 20% and ≤ 70% by weight, based on the total weight of the composition. This calculation includes the proportion of solvent introduced into the composition with the polyurethane urea solution according to the invention in the solvent content reported here.

In a further embodiment of the invention, the physiologically acceptable medium of the composition according to the invention comprises water. The water used in the composition according to the invention may be a blossom water, pure demineralized water, mineral water, thermal water and/or seawater.

More preferably, however, the composition according to the invention contains ≤ 10% by weight of water, more preferably ≤ 5% by weight of water, based on the total weight of the composition. Most preferably, the nail varnish composition is purely solvent-based and does not contain any water.

In order to improve the film-forming properties of the nail varnishes according to the invention, it is possible to use film-forming aids. The film-forming aid may be selected from the group of the plasticizers and/or coalescing agents.

Plasticizers used are advantageously plasticizers and/or plasticizing resins having a number-average molecular weight of less than 1500 g/mol, in order to achieve the desired mechanical properties. Suitable plasticizers are, for example, glycols and esters and ethers thereof, esters of acids, especially carboxylic acids, such as citrates, adipates, carbonates, tartrates, phosphates or sebacates, ethoxylated derivatives such as ethoxylated oils and/or mixtures thereof. For example, suitable plasticizers are diethylene glycol ethyl ether, diethylene glycol methyl ether, diethylene glycol n-butyl ether or diethylene glycol hexyl ether, ethylene glycol ethyl ether, ethylene glycol butyl ether, ethylene glycol hexyl ether, propylene glycol phenyl ether, propylene glycol diacetate, dipropylene glycol ethyl ether, tripropylene glycol methyl ether, diethylene glycol methyl ether, propylene glycol butyl diglycol solution, tributyl phosphate, tributoxyethyl phosphate, tricresyl phosphate, triphenyl phosphate, glycerol triacetate, butyl stearate, butyl glycolate, benzyl benzoate, butyl acetyltricinoleate, glyceryl acetyltricinoleate, dibutyl phthalate, diisobutyl phthalate, dioctyl phthalate, dimethoxyethyl phthalate, diethyl phthalate, diamyl phthalate, triethyl citrate, tributyl citrate, tributyl acetylcitrates, triethyl acetylcitrates, tri(2-ethylhexyl)acetyl-, dibutyl tartrate, triacetin, camphor, trimethylpentanyl diisobutyrate, triethylhexanoïne, sucrose benzoate, dibutyl adipate, diisobutyl adipate, diisopropyl adipate, dipropylene glycol dibenzoate and/or mixtures thereof.

Particular resins having low molecular weight may likewise be used as plasticizers and are considered to be external plasticizers since they plasticize the systems without dissolving the cellulosic film-forming resins. Advantageous plasticizers that should be mentioned are adipate polyesters, sebacate polyesters or butyl acrylate resins.

Suitable plasticizers are, for example, also the plasticizers containing carbonate groups, as described in WO2015/022438, US 2010/0158835 A1, WO 03/094870 A.

Especially suitable are plasticizers selected from polycarbonate polyols, polyester carbonate polyols and/or polyether carbonate polyols or mixtures thereof, for example the Bayer MaterialScience AG products available under the Desmophen® C 2100, Desmophen® C 2200, Desmophen® C XP 2613, Desmophen® C 3100 XP, Desmophen® C 3200 XP and Desmophen® C XP 2716 trade names.

The proportion of plasticizers in the composition according to the invention is preferably in the range of ≥ 0% and ≤ 25% by weight, more preferably ≥ 0.5% and ≤ 15% by weight and most preferably ≥ 1% and ≤ 5% by weight, based on the total weight of the composition.

The preferred coalescing agents are, for example, propylene glycol ethers, for example propylene glycol n-butyl ether, propylene glycol t-butyl ether, propylene glycol n-propyl ether, propylene glycol phenyl ether, dipropylene glycol ethers, for example dipropylene glycol n-butyl ether, dipropylene glycol methyl ether, dipropylene glycol t-butyl ether, dipropylene glycol n-propyl ether, propylene glycol methyl ether acetate, propylene glycol diacetate, methyl lactate, ethyl lactate, isopropyl lactate, butyl lactates and mixtures thereof.

The proportion of coalescing agents in the composition according to the invention may, for example, be in the range of ≥ 0% and ≤ 10% by weight and preferably ≥ 0.1% and ≤ 8% by weight, based on the total weight of the composition.

The composition according to the invention may comprise a special-effect constituent. The constituents mentioned may especially have a colouring effect or else provide other effects, such as sparkle and/or metallic effects. Preferably, the composition according to the invention comprises at least one dye which is preferably selected from the group of lipophilic dyes, hydrophilic dyes, pigments, paillettes and mother of pearl.

Particularly advantageously in accordance with the invention, the concentration of the special-effect constituents is ≥ 0% and ≤ 50% by weight, particularly advantageously ≥ 0.1% and ≤ 30% by weight, very particularly advantageously ≥ 0.5% and ≤ 15% by weight, based in each case on the total weight of the composition.

For example, it is possible to use lipophilic dyes, such as Sudan I (yellow), Sudan II (orange), Sudan III (red), Sudan IV (scarlet), DC Red 17, DC Green 6, β-carotene, soya oil, DC Yellow 11, DC Violet 2, DC Orange 5 and DC Yellow 10.

For example, it is possible to use hydrophilic dyes, such as beetroot juice and methylene blue.

The pigments may in principle be any inorganic or organic pigments which are used in cosmetic or dermatological compositions. The pigments used in accordance with the invention may, for example, be white or coloured, and they may be encased or coated with a hydrophobic coating composition or be uncoated.

Advantageously, the pigments are selected from the group of the metal oxides, such as the oxides of iron (especially the oxides that are yellow, red, brown or black in colour), titanium dioxide, zinc oxide, cerium oxide, zirconium oxide, chromium oxide; manganese violet, ultramarine blue, Prussian blue, ultramarine and iron blue, bismuth oxide chloride, mother of pearl, mica pigments coated with titanium or bismuth oxide chloride, coloured pearlescent pigments, for example titanium-mica pigments comprising iron oxides, titanium-mica pigments, especially comprising iron blue or chromium oxide, titanium-mica pigments comprising an organic pigment of the aforementioned type, and pearlescent pigments based on bismuth oxide chloride, carbon black, the pigments of the D&C type, such as D&C Red N° 5, 6, 7, 10, 11, 12, 13, 34, D&C Yellow Lake N°5 and D&C Red Lake N° 2, and the coating materials based on cochineal red, barium, strontium, calcium and aluminium, and mixtures thereof.

To adjust rheological properties, it is possible to add thickeners as additives. Particularly advantageously in accordance with the invention, the concentration of thickeners is ≥ 0% and ≤ 20% by weight, particularly advantageously ≥ 0.1% and ≤ 15% by weight, very particularly advantageously ≥ 0.5% and ≤ 10% by weight, based in each case on the total weight of the composition.

Advantageous thickeners are:
- Thickening polymers of natural origin, for example based on cellulose, guar gum, xanthan, scleroglucan, gellan gum, rhamsan and karaya gum, alginates, maltodextrin, starch and derivatives thereof, carob seed flour, hyaluronic acid, carrageenan, organomodified clays such as organomodified bentonites, stearalkonium bentonites, organomodified hectorites, stearalkonium hectorites, or organomodified montmorillonite, hydrophobic fumed silica wherein the silanol groups have been replaced by trimethylsiloxy groups (AEROSIL® R812 from Evonik) or by dimethylsiloxy groups or polydimethylsiloxane (AEROSIL® R972, AEROSIL® R974 from Evonik, CAB-O-SIL® TS-530, CAB-O-SIL® TS-610, "CAB-O-SIL® TS-720 from Cabot), polysaccharide alkyl ethers (as described in EP 898958-A).
- Nonionic associative polymers, for example based on polyethylene glycols and derivatives thereof, or polyurethanes.

Particularly advantageous thickeners are organomodified clays based on bentonites, hectorites and laponites, associative polymers, cellulose and derivatives thereof, for example hydroxy ethyl cellulose and carboxymethyl cellulose.

The nail vanishes according to the invention may contain fillers. Fillers are understood to mean colourless or white, mineral or synthetic, lamellar, spherical or elongated inert particulate fillers which are insoluble in the medium of the nail varnishes according to the invention, which modify, for example, the rheological properties and the texture of the formulations.

The fillers may be present in the composition according to the invention, for example, in an amount of ≥ 0% and ≤ 50% by weight, based on the total weight of the composition, and preferably of ≥ 0.1% and ≤ 30% by weight.

Advantageous particulate fillers in the context of the present invention are talc, mica, silicon dioxide, kaolin, starch and derivatives thereof (for example tapioca starch, di-starch phosphate, aluminium starch or sodium starch octenylsuccinate and the like), fumed silica, pigments having neither principally UV filter action nor colouring action (for example boron nitride, etc.), boron nitride, calcium carbonate, dicalcium phosphate, magnesium carbonate, magnesium hydrogencarbonate, hydroxyapatites, microcrystalline cellulose, powders of synthetic polymers such as polyamides (for example the polymers available under the "Nylon®" trade name), polyethylene, poly-β-alanine, polytetrafluoroethylene ("Teflon®"), polyacrylate, polyurethane, lauroyllysines, silicone resin (for example the polymers available under the "Tospearl®" trade name from Momentive Performance Materials), polyorganosiloxane elastomer, hollow particles of polyvinylidlene/acrylonitriles (Expancel® from Akzo Nobel) or hollow particles of silicon dioxide (Silica Beads® from MAPRECOS), glass or ceramic microcapsules, and metal soaps derived from organic carboxylic acids having 8 to 22 carbon atoms, preferably 12 to 18 carbon atoms, for example zinc stearate, magnesium stearate or lithium stearate, zinc laurate, magnesium myristate.

A particular advantage of the nail varnishes according to the invention is that the fingernail care additives can also be incorporated into the varnish. Suitable examples are vitamins B5, E and C and derivatives thereof, and also dimethyloxobenzodioxasilanes, calcium chloride, calcium pantothenate, panthenol, proteins, ceramides, myrrhs, plant extracts, amino acid oils, for example cysteine and salts and derivatives thereof, cysteine, glutathione, biotin, urea and dimethylurea, alpha-hydroxy acids such as citric acid and ascorbic acid, UV stabilizers such as benzophenone-1, benzophenone-3, benzyl salicylate, etocrylene, drometrizole, butyl methoxydibenzoylmethane, and hardening additives such as formaldehyde and hydrolysates formed from chitin and/or keratin. Antimycotic additives are also possible.

The care additives may be present in the composition according to the invention, for example, in an amount of ≥ 0% and ≤ 5% by weight, based on the total weight of the composition, and preferably of ≥ 0.1% and ≤ 3% by weight.

In addition, the composition according to the invention may also contain additives customary for nail varnish compositions, for example antioxidants, light stabilizers, wetting agents, emulsifiers, dispersants, stabilizers, defoamers, levelling agents, preservatives, moisturizing substances, perfume, free-radical scavengers, neutralizers, oils, waxes and active ingredients. According to the desired profile of properties and end use of the composition according to the invention, up to 90% by weight, based on total dry matter, of these additives (based on the sum of all the additives) may be present in the end product. Total dry matter is understood to mean all the non-volatile components of the nail varnish composition.

Preferably, the nail varnish composition according to the invention further comprises organic solvents, and also plasticizers and/or special-effect constituents.

More preferably, the nail varnish composition according to the invention comprises organic solvents, rheologically modifying additives, and also plasticizers and/or special-effect constituents.

In a preferred embodiment of the invention, the nail varnish composition according to the invention comprises the polyurethane urea solution used in accordance with the invention, organic solvents special-effect constituents, plasticizers and optionally further film formers and/or additives customary in nail varnishes, such as especially rheologically modifying additives.

In an additionally preferred embodiment of the invention, the nail varnish composition comprises
A) ≥ 0% and ≤ 10% by weight of water,
B) ≥ 0.2% and ≤ 40% by weight of the polyurethane urea used in accordance with the invention (as active substance without solvent),
C) ≥ 10% and ≤ 95% by weight of organic solvents,
D) ≥ 0% and ≤ 30% by weight of different film formers from B),
E) ≥ 0% and ≤ 25% by weight of plasticizers,
F) ≥ 0% and ≤ 30% by weight of special-effect constituents,
G) ≥ 0% and ≤ 60% by weight of further additives customary in nail varnishes,
based in each case on the total mass of the nail varnish composition, and where components A) to G) add up to 100% by weight, and where the polyurethane urea is dissolved in a portion or the whole amount of organic solvent. This calculation includes the proportion of solvent introduced into the composition with the polyurethane urea solution according to the invention in the solvent content C) reported here. The figures for B) relate purely to the polyurethane urea present in the solution as active substance.

More preferably, the nail varnish composition consists of components A) to G).

The further additives customary in nail varnishes are understood to mean especially coalescing agents, thickeners, fillers and/or nail care additives.

In a particularly preferred embodiment of the invention, the nail varnish composition comprises
A) ≥ 0% and ≤ 5% by weight of water,
B) ≥ 0.5% and ≤ 30% by weight of the polyurethane urea used in accordance with the invention (as active substance without solvent),
C) ≥ 15% and ≤ 95% by weight of organic solvents,
D) ≥ 0% and ≤ 20% by weight of different film formers from B),
E) ≥ 0.5% and ≤ 15% by weight of plasticizers,
F) ≥ 0.1% and ≤ 30% by weight of special-effect constituents,
G) ≥ 0% and ≤ 60% by weight of further additives customary in nail varnishes,
based in each case on the total mass of the nail varnish composition, and where components A) to G) add up to 100% by weight, and where the polyurethane urea is dissolved in a portion or the whole amount of organic solvent. This calculation includes the proportion of solvent introduced into the composition with the polyurethane urea solution according to the invention in the solvent content C) reported here. The figures for B) relate purely to the polyurethane urea present in the solution as active substance.

Especially preferably, the nail varnish composition consists of components A) to G).

The nail varnish compositions according to the invention may also be systems curable by UV radiation. However, they are preferably non-UV-curable systems.

The process for the production of the nail varnish composition is preferably carried out by mixing all components with the polyurethane urea solution at temperatures ≥ 10 and ≤ 85°C, preferably ≥ 20 and ≤ 75°C. The different components may be added at once, but also stepwise. The temperature ranges may be different for each step of the stepwise mixing process.

In one embodiment of the invention the mixing is carried out with a dissolver disc for 5 to 60 min.

After mixing the components can be milled with a pearl mill for 10 to 120 min. This is especially helpful if the composition comprises special-effect constituents.

The nail varnish composition according to the invention is preferably dispensed into vessels having a capacity of ≤ 50 ml, preferably ≤ 20 ml.

The invention also provides for the use of the polyurethane urea solution used in accordance with the invention for cosmetic coating of nails.

The invention further provides a process for producing a cosmetic coating on nails using the nail varnish compositions according to the invention, wherein the nail varnish composition is applied to nails.

Advantageously, in the process according to the invention, the nail varnish compositions according to the invention remain at least partly on the nails.

The nail varnish composition is applied to the fingernails and/or toenails with an aid. The aid is especially a fine brush or an application aid having a similar effect. After the application, a film preferably forms on the nail. This film then preferably hardens. The film preferably hardens at 23°C within ≤ 20 min. The drying time at room temperature was determined after application of the composition to a glass plate (120 µm wet) by means of a drying time recorder from BYK Gardner GmbH, Germany to ASTM D5895. The drying time "to the touch" was ascertained.

The film can be cured without aids, but also with the aid, for example, of a UV lamp.

The coating obtainable from the nail varnish composition according to the invention preferably has a pendulum hardness of ≥ 40 König/s and ≤ 250 König/s, preferably ≥ 50 König/s and ≤ 200 König/s. The pendulum hardnesses are determined on a Konig Pendulum Hardness Tester from BYK Gardner GmbH, Germany to DIN EN ISO 1522, after drawdown (240 µm wet) and drying of the respective composition overnight (16 hours) at 23°C and 50% relatively air humidity on a glass plate. In each case, the mean was formed from 3 measurements.

In addition, the coating obtainable from the nail varnish formulation according to the invention preferably has a gloss of ≥ 70 and ≤ 99, more preferably of ≥ 80 and ≤ 98. Gloss is determined to DIN 67530 by means of a "micro-haze plus" gloss meter from BYK Gardner GmbH, Germany, after drawdown (200 µm wet) and drying at 23°C of the respective composition after 24 hours on a black/white Leneta card. Gloss is measured at an angle of 60°.

In addition, the coating obtainable from the nail varnish formulation according to the invention is preferably resistant to water, meaning more particularly that the hardened coating film obtained, after 4 h in water and 40°C, is still complete and smooth and still adheres to the substrate to which it has been applied; more preferably, the film is then still complete and clear and still adheres to the substrate to which it has been applied. To measure water resistance, glass plates, after application of the composition (240 µm) and drying at 23°C and 50% relatively air humidity for 24 hours, were immersed into a water bath at 40°C over a period of 4 hours. Water resistance was determined qualitatively.

One advantage of the invention is that the films obtained by using the nail varnish composition show a good water resistance, but at the same time can be easily removed using alcohol-based nail varnish removers. Those nail varnish removers are more sensitive to nails and skin compared to those based on acetone or ethyl acetate.

Alcohol-based nail varnish removers are for example based on monohydroxy-functional alcohols, such as ethanol or isopropanol.

The invention further provides a kit comprising a nail varnish composition according to the invention and an alcohol-based nail varnish remover.

The nail varnish an the nail varnish remover should be in different containers or at least in one container having separate compartments for each of the substances.

The present invention is elucidated by the following examples.

### Examples:

Unless indicated otherwise, all percentages are based on weight.

Unless stated otherwise, all analytical measurements relate to temperatures of 23°C.

The solids contents (nonvolatile component) were determined to DIN EN ISO 3251.

Unless explicitly mentioned otherwise, NCO contents were determined by volumetric means to DIN EN ISO 11909.

The check for free NCO groups was conducted by means of IR spectroscopy (band at 2260 cm⁻¹).

The viscosities reported were determined by means of rotary viscometry to DIN 53019 at 23°C with a rotary viscometer from Anton Paar Germany GmbH, Ostfildern, DE.

The number-average molecular weight was determined by gel permeation chromatography (GPC) in tetrahydrofuran at 23°C. The procedure is according to DIN 55672-1: "Gel permeation chromatography, Part 1 - Tetrahydrofuran as eluent" (SECurity GPC System from PSS Polymer Service, flow rate 1.0 ml/min; columns: 2×PSS SDV linear M, 8×300 mm, 5 µm; RID detector). Polystyrene samples of known molar mass are used for calibration. The number-average molecular weight is calculated with software support. Baseline points and evaluation limits are fixed in accordance with DIN 55672 Part 1.

Turbidity values [NTU] were determined by a scattered light measurement at a 90° angle (nephelometry) at a measurement radiation wavelength of 860 nm in accordance with DIN EN ISO 7027, conducted at 23°C with a model 2100AN laboratory turbidimeter from HACH LANGE GmbH, Berlin, Germany.

### Substances and abbreviations used:

- PolyTHF® 2000:: polytetramethylene glycol polyol, OH number 56 mg KOH/g, number-average molecular weight 2000 g/mol (BASF AG, Ludwigshafen, DE)
- PolyTHF® 1000:: polytetramethylene glycol polyol, OH number 112 mg KOH/g, number-average molecular weight 1000 g/mol (BASF AG, Ludwigshafen, DE)
- Ethanol: Unless stated otherwise, MEK-denatured ethanol from Nordbrand, Norhausen, DE was used.

Isocyanates and the further polymeric polyols were used from Bayer MaterialScience AG, Leverkusen, DE. Further chemicals were purchased from Sigma-Aldrich Chemie GmbH, Taufkirchen, DE. The raw materials, unless stated otherwise, were used without further purification or pretreatment.

### Example 1: Preparation of a polyurethane solution in ethanol (inventive)

150 g of PolyTHF® 2000 and 37.50 g of PolyTHF® 1000 were dewatered under membrane pump vacuum at 100°C for one hour in a standard stirrer apparatus and then initially charged at 80°C under nitrogen. Then 75.06 g of isophorone diisocyanate were added at 80°C within 5 min and stirring at 110°C was continued (about 3 hours) until the NCO value had gone below the theoretical value. The prepolymer was cooled to 40°C and it was dissolved in 630.4 g of ethanol (denatured with diethyl phthalate) and then the temperature was reduced to 15°C. Then a solution of 37.6 g of methylenebis(4-aminocyclohexane) and 270 g of ethanol (denatured with diethyl phthalate) was metered in within 30 min; after a further 30 minutes at 20°C, isocyanate groups were still detectable by IR spectroscopy. Stirring of the mixture was continued at 23°C for about 16 hours until no free isocyanate groups were detectable any longer by IR spectroscopy.

The resultant clear, storage-stable solution had the following properties:

| | |
|---|---|
| Solids content: | 23 % |
| Viscosity (viscometer, 23°C): | 280 mPas |
| Turbidity value: | 1.2 NTU |

Films obtained from the polyurethane urea solutions were at 23°C soluble in ethanol or isopropanol, but non-soluble in acetone, water or ethyl acetate.

### Example 2: Preparation of a polyurethane solution in ethanol (inventive)

300 g of PolyTHF® 1000 were dewatered under membrane pump vacuum at 100°C for one hour in a standard stirrer apparatus and then initially charged at 80°C under nitrogen. Then 133.44 g of isophorone diisocyanate were added at 80°C within 5 min and stirring at 110°C was continued (about 3 hours) until the NCO value had gone below the theoretical value. The prepolymer was cooled to 40°C and it was dissolved in 517 g of ethanol (denatured with MEK) and then the temperature was reduced to 16°C. Then a solution of 58.8 g of methylenebis(4-aminocyclohexane) and 222 g of ethanol (denatured with MEK) was metered in within 30 min; then a further 410 g of ethanol were added. Stirring was continued until no free isocyanate groups were detectable any longer by IR spectroscopy.

The resultant clear, storage-stable solution had the following properties:

| | |
|---|---|
| Solids content: | 30.2 % |
| Viscosity (viscometer, 23°C): | 85000 mPas |

Films obtained from the polyurethane urea solutions were at 23°C soluble in ethanol or isopropanol, but non-soluble in acetone, water or ethyl acetate.

### Example 3: Preparation of a polyurethane solution in ethanol (inventive)

300 g of PolyTHF® 1000 were dewatered under membrane pump vacuum at 100°C for one hour in a standard stirrer apparatus and then initially charged at 80°C under nitrogen. Then 133.44 g of isophorone diisocyanate were added at 80°C within 5 min and stirring at 110°C was continued (about 3 hours) until the NCO value had gone below the theoretical value. The prepolymer was cooled to 40°C and it was dissolved in 498 g of ethanol (denatured with MEK) and then the temperature was reduced to 16°C. Then a solution of 40.9 g of isophoronediamine and 213 g of ethanol (denatured with MEK) was metered in within 30 min; then a further 7.15 g of ethanol isophoronediamine were added. Stirring was continued until no free isocyanate groups were detectable any longer by IR spectroscopy.

The resultant clear, storage-stable solution had the following properties:

| | |
|---|---|
| Solids content: | 40.8 % |
| Viscosity (viscometer, 23°C): | 3850 mPas |

Films obtained from the polyurethane urea solutions were at 23°C soluble in ethanol or isopropanol, but non-soluble in acetone, water or ethyl acetate.

### Example 4: Preparation of a polyurethane solution in ethanol (inventive)

250 g of PolyTHF® 2000 and 62.5 g of PolyTHF® 1000 were dewatered under membrane pump vacuum at 100°C for one hour in a standard stirrer apparatus and then initially charged at 80°C under nitrogen. Then 83.4 g of isophorone diisocyanate were added at 80°C within 5 min and stirring at 110°C was continued (about 3 hours) until the NCO value had gone below the theoretical value. The prepolymer was cooled to 40°C and it was dissolved in 425 g of ethanol and then the temperature was reduced to 18°C. Then a solution of 9.0 g of ethylenediamine and 180 g of ethanol was metered in within 30 min. A further 0.83 g of ethylenediamine was added and then stirring was continued until no free isocyanate groups were detectable any longer by IR spectroscopy.

The resultant clear, storage-stable solution had the following properties:

| | |
|---|---|
| Solids content: | 40.7 % |
| Viscosity (viscometer, 23°C): | 34600 mPas |

Films obtained from the polyurethane urea solutions were at 23°C soluble in ethanol or isopropanol, but non-soluble in acetone, water or ethyl acetate.

### Example 5: Preparation of a polyurethane solution in ethanol (inventive)

211 g of PolyTHF® 2000 and 52.7 g of PolyTHF® 1000 were dewatered under membrane pump vacuum at 100°C for one hour in a standard stirrer apparatus, then 5.4 g of neopentyl glycol were added and the mixture was subsequently initially charged at 80°C under nitrogen. Then 93.4 g of isophorone diisocyanate were added at 80°C within 5 min and stirring at 110°C was continued (about 3 hours) until the NCO value had gone below the theoretical value. The prepolymer was cooled to 40°C and it was dissolved in 420 g of ethanol (denatured with diethyl phthalate) and then the temperature was reduced to 17°C. Then a solution of 35.3 g of methylenebis(4-aminocyclohexane) and 180 g of ethanol (denatured with diethyl phthalate) was metered in within 30 min. A further 0.67 g of methylenebis(4-aminocyclohexane) was added and then stirring was continued until no free isocyanate groups were detectable any longer by IR spectroscopy.

The resultant clear, storage-stable solution had the following properties:

| | |
|---|---|
| Solids content: | 40.5 % |
| Viscosity (viscometer, 23°C): | 7060 mPas |

Films obtained from the polyurethane urea solutions were at 23°C soluble in ethanol or isopropanol, but non-soluble in acetone, water or ethyl acetate.

### Example 6: Preparation of a polyurethane solution in ethanol (inventive)

226.2 g of polypropylene glycol having a number-average molecular weight of 2000 g/mol and 62.5 g of polypropylene glycol having a number-average molecular weight of 1000 g/mol were dewatered under membrane pump vacuum at 100°C for one hour in a standard stirrer apparatus, then the mixture was initially charged at 80°C under nitrogen. Then 83.4 g of isophorone diisocyanate were added at 80°C within 5 min and the mixture was stirred at 120°C for 6 hours until the NCO value had gone below the theoretical value. The prepolymer was cooled to 40°C and it was dissolved in 280 g of ethanol and then the temperature was reduced to 18°C. Then a solution of 34.1 g of methylenebis(4-aminocyclohexane) and 120 g of ethanol was metered in within 30 min. A further 4.5 g of methylenebis(4-aminocyclohexane) were added and then stirring was continued until no free isocyanate groups were detectable any longer by IR spectroscopy.

The resultant clear, storage-stable solution had the following properties:

| | |
|---|---|
| Solids content: | 49.8 % |
| Viscosity (viscometer, 23°C): | 1100 mPas |

Films obtained from the polyurethane urea solutions were at 23 °C soluble in ethanol or isopropanol, but non-soluble in acetone, water or ethyl acetate.

### Example 7: Preparation of a polyurethane solution in ethanol (inventive)

160 g of PolyTHF® 2000 and 40.0 g of PolyTHF® 1000 were dewatered under membrane pump vacuum at 100°C for one hour in a standard stirrer apparatus and then initially charged at 80°C under nitrogen. Then 62.9 g of bis(4,4'-isocyanatocyclohexyl)methane were added at 80°C within 5 min and stirring at 110°C was continued (about 3 hours) until the NCO value had gone below the theoretical value. The prepolymer was cooled to 40°C and it was dissolved in 595 g of ethanol and then the temperature was reduced to 19°C. Then a solution of 20.2 g of methylenebis(4-aminocyclohexane) and 255 g of ethanol was metered in within 30 min. A further 4.5 g of methylenebis(4-aminocyclohexane) were added and then stirring was continued until no free isocyanate groups were detectable any longer by IR spectroscopy.

The resultant clear, storage-stable solution had the following properties:

| | |
|---|---|
| Solids content: | 25.2 % |
| Viscosity (viscometer, 23°C): | 3400 mPas |

Films obtained from the polyurethane urea solutions were at 23 °C soluble in ethanol or isopropanol, but non-soluble in acetone, water or ethyl acetate.

### Example 8: Preparation of a polyurethane solution in ethanol (inventive)

200 g of PolyTHF® 2000 and 50.0 g of PolyTHF® 1000 were dewatered under membrane pump vacuum at 100°C for one hour in a standard stirrer apparatus and then initially charged at 70°C under nitrogen. Then 50.4 g of hexamethylene diisocyanate were added at 70°C within 5 min and stirring at 110°C was continued (about 3 hours) until the NCO value had gone below the theoretical value. The prepolymer was cooled to 40°C and it was dissolved in 340 g of ethanol and then the temperature was reduced to 18°C. Because of the high viscosity, a further 270 g of ethanol were added, then a solution of 25.2 g of methylenebis(4-aminocyclohexane) and 150 g of ethanol was metered in within 30 min. Then stirring was continued until it was no longer possible to detect any free isocyanate groups by IR spectroscopy.

The resultant clear, storage-stable solution had the following properties:

| | |
|---|---|
| Solids content: | 30.1 % |
| Viscosity (viscometer, 23°C): | 15500 mPas |

Films obtained from the polyurethane urea solutions were at 23°C soluble in ethanol or isopropanol, but non-soluble in acetone, water or ethyl acetate.

### Comparative example 9: Attempted preparation of a polyurethane urea solution in ethanol

100 g of PolyTHF® 2000, 25.0 g of PolyTHF® 1000 and 127.5 g of a linear difunctional amorphous polyester diol based on adipic acid, hexane-1,6-diol and neopentyl glycol and having a number-average molecular weight of 1700 g/mol were dewatered under membrane pump vacuum at 100°C for one hour in a standard stirrer apparatus and then initially charged at 80°C under nitrogen. Then 66.7 g of isophorone diisocyanate were added at 80°C within 5 min and stirring at 110°C was continued (about 3 hours) until the NCO value had gone below the theoretical value. The prepolymer was cooled to 40°C and it was dissolved in 720 g of ethanol, although the product did not dissolve completely, and then the temperature was reduced to 17°C. Then a solution of 25.2 g of methylenebis(4-aminocyclohexane) and 310 g of ethanol was metered in within 30 min, which gave rise to white turbidity. Then stirring was continued, which did not form a stable solution but resulted in a biphasic mixture from which the solid phase settled out.

### Comparative example 10: Attempted preparation of a polyurethane urea solution in ethanol

200 g of a linear difunctional polycarbonate diol based on hexane-1,6-diol, having a number-average molecular weight of 2000 g/mol, and 50 g of a linear difunctional polycarbonate diol based on hexane-1,6-diol, having a number-average molecular weight of 1000 g/mol, were dewatered under membrane pump vacuum at 100°C for one hour in a standard stirrer apparatus and then initially charged at 80°C under nitrogen. Then 66.7 g of isophorone diisocyanate were added at 80°C within 5 min and stirring at 110°C was continued (about 3 hours) until the NCO value had gone below the theoretical value. The prepolymer was cooled to 40°C and it was dissolved in 720 g of ethanol, although the product did not dissolve completely, and then the temperature was reduced to 17°C. Then a solution of 25.2 g of methylenebis(4-aminocyclohexane) and 310 g of ethanol was metered in within 30 min, which gave rise to a biphasic mixture. Then stirring was continued, which did not form a stable solution but resulted in a biphasic mixture from which the solid phase settled out.

### Applied examples:

### Substances used:

- Collodion:: mixture of 50% by weight of butyl acetate, 26% by weight of ethyl acetate and 24% by weight of nitrocellulose (the nitrocellulose used contains 17% by weight of isopropanol).
- Baycusan C1008:: aqueous dispersion of an ionically hydrophilized polyurethane urea, commercial product from Bayer MaterialScience AG, Leverkusen; solids content: 30% by weight
- Baycusan C1004:: aqueous dispersion of an ionically hydrophilized polyurethane urea, commercial product from Bayer MaterialScience AG, Leverkusen; solids content: 40% by weight

### Example 11: Comparison: Aqueous polyurethane urea dispersion and polyurethane urea solution

A sufficient amount of polyurethane urea solution from Example 1, Baycusan C1004 or Baycusan C1008 in each case was mixed with butyl acetate, ethyl acetate and collodion (50% by weight of collodion plus butyl acetate made up to 100% by weight), so as to give a proportion of active polyurethane urea (solids content) of 2% by weight, based on the overall mixture. Each mixture was stirred at room temperature for 10 min. Then miscibility was assessed visually. Table 1 shows the appearance of each mixture. It was shown clearly that the aqueous polyurethane dispersions are incompatible with the important nail varnish constituents collodion, and butyl or ethyl acetate, while the polyurethane urea solution used in accordance with the invention leads to clear solutions.

**Table 1:**

| Constituents | Collodion (50% by weight + butyl acetate) | Butyl acetate | Ethyl acetate |
|---|---|---|---|
| Baycusan C1008 | white, turbid | sedimentation, flocculation | slightly turbid, flocculant |
| Baycusan C1004 | slightly turbid, flocculant | sedimentation, flocculation | slightly turbid, flocculant |
| Polyurethane urea solution from Example 1 | clear solution | clear solution | clear solution |

### Example 12: Inventive nail varnish composition comprising polyurethane urea solution as secondary film former

50% by weight of collodion, 5.3% by weight of butyl acetate, 39.7% by weight of the solution from Example 1 (corresponding to 10% by weight of polyurethane urea as active substance) and 5% by weight of acetyl tributyl citrate were mixed at room temperature in the sequence specified. A clear composition was obtained. The resultant nail varnish composition was examined for drying time, gloss and hardness.

The nail varnish composition exhibited a pendulum hardness of 35 sec, a drying time of 142 sec, and a gloss of 87° (white) and 83° (black).

The drying time at room temperature was determined after application of the composition to a glass plate (120 µm wet) by means of a drying time recorder from BYK Gardner GmbH, Germany to ASTM D5895. The drying time "to the touch" was ascertained.

The pendulum hardnesses were determined on a Konig Pendulum Hardness Tester from BYK Gardner GmbH, Germany to DIN EN ISO 1522, after drawdown (240 µm wet) and drying of the respective composition overnight (16 hours) at 23°C and 50% relatively air humidity on a glass plate. In each case, the mean was formed from 3 measurements.

Gloss was determined to DIN 67530 by means of a "micro-haze plus" gloss meter from BYK Gardner GmbH, Germany, after drawdown (200 µm wet) and drying at 23°C of the respective composition after 24 hours on a black/white Leneta card. Gloss is measured at an angle of 60°.

### Example 13: Nail varnishes comprising polyurethane urea solution according to the invention as primary film former

**Table 2:**

| **PHASE** | **INCI** | **TRADE NAME** | **SUPPLIER** | **FORMULATION WITH BAYCUSAN C 1004 WT.-%** | **FORMULATION WITH POLYURETHANE-UREA SOLUTION ACCORDING TO THE INVENTION WT.-%** |
|---|---|---|---|---|---|
| **A** | Water | | | To 100 | - |
| | Hydroxyeth ylcellulose | Natrosol 250HHR | Ashland | 0.85 | - |
| **B** | Polysorbate 60 | Tween 80 | local | 0.30 | - |
| | Cyclomethic one | Dow Corning 245 Fluid | Dow corning | 0.10 | - |
| | Mica (and) Titanium Dioxide (and) Carmine | Colorona Carmine Red | Merck | 0.15 | 0.15 |
| | Calcium Aluminum Borosilicate (and) Titanium Dioxide (and) Silica (and) Tin Oxide | Ronastar Silver | Merck | 3.00 | 3.00 |
| **C** | Filmformer Baycusan C 1004 or Polyurethan e urea according to the invention | | Bayer MaterialSc ience AG | 60.00 | 96.85 |

### Preparation of formulation with Baycusan C 1004 according to table 2:

The Hydroxyethycellulose was dissolved in water at 70°C under stirring (about 50 rpm). Afterwards the formulation was cooled down to room temperature. At 50°C, the phase B was added to phase A under continuous mixing. At 30°C, phase C was added to phase A+B under continuous mixing. At room temperature, the formulation was moderately homogenized during 10 min. at 3000 rpm.

### Preparation of formulation with polyurethanure solution according to the invention:

At room temperature, phase B was dispersed in the polyurethanepolyurea solution according to the invention (phase C) under stirring (about 50 rpm). When a homogeneous mixture was obtained, the formulation was moderately homogenized further during 10 min at 3000 rpm.

### Evaluation results:

**Table 3:**

| | **NAIL POLISH WITH BAYCUSAN C 1004** | **NAIL POLISH WITH POLYURETHANE UREA SOLUTION ACCORDING TO THE INVENTION** |
|---|---|---|
| % film left after adhesion test | 100 | 10 |
| % film left after water resistance test | 75 | 100 |

Adhesion test : Nail polish formulation was applied 100 µm wet on glass plate. After drying at room temperature, an commercial adhesive tape was applied on the film and then peeled with the same strength. The quantity of film left after the test is determined.

Water resistance: Nail polish formulation was applied 100 µm wet on glass plate. After drying at room temperature, the glass plate was put 10 min. under running water. The quantity of film left after the test is analyzed.

The tests show that the nail polish formulations according to the invention show an improved water resistance and can be easily peeled off in one piece compared to formulations containing film formers according to the state of the art.

## Claims

1. Process for producing a nail varnish composition, **characterized in that** at least one polyurethane urea which has no ionically hydrophilizing groups and has been dissolved in a solvent or solvent mixture is used, the solvent consisting exclusively of one or more monohydroxy-functional alcohols or being a solvent mixture consisting of exclusively organic solvents and containing ≥ 50% by weight, based on the total mass of the solvent mixture, of at least one monohydroxy-functional alcohol,
where the polyurethane urea has been formed from
a) at least one aliphatic, araliphatic and/or cycloaliphatic diisocyanate,
b) at least one polyether polyol having a number-average molecular weight Mn of ≥ 400 and ≤ 6000 g/mol and a hydroxyl functionality of ≥ 1.5 and ≤ 4,
c) at least one amino-functional compound having at least two isocyanate-reactive amino groups,
d) optionally at least one alcohol having at least two hydroxyl groups and a molar mass of ≥ 60 and ≤ 399 g/mol,
e) optionally at least one compound having a group reactive toward isocyanate groups and
f) optionally ≤ 20% by weight, based on the total mass of the polyurethane urea, of at least one different polyol from b) having a number-average molecular weight Mn of ≥ 500 and ≤ 6000 g/mol and a hydroxyl functionality of ≥ 1.5 and ≤ 4.

2. Process according to Claim 1, **characterized in that** the polyurethane urea does not have any hydrophilizing groups.

3. Process according to Claim 1 or 2, **characterized in that** component b) is selected from poly(tetramethylene glycol) polyether polyols.

4. Process according to any of Claims 1 to 3, **characterized in that** component b) has a number-average molecular weight Mn of ≥ 500 and ≤ 2500 g/mol and a hydroxyl functionality of ≥ 1.9 and ≤ 3.

5. Process according to any of Claims 1 to 4, **characterized in that** component a) is selected from aliphatic, araliphatic and/or cycloaliphatic diisocyanates having at least one isocyanate group bonded to a secondary and/or tertiary carbon atom.

6. Process according to any of Claims 1 to 5, **characterized in that** component a) is selected from IPDI and/or H12-MDI.

7. Process according to any of Claims 1 to 6, **characterized in that** component c) is selected from amines having at least two amino groups bonded to primary and/or secondary carbon atoms.

8. Process according to any of Claims 1 to 7, **characterized in that** component c) is selected from symmetrical diamines.

9. Process according to any of Claims 1 to 8, **characterized in that** component c) is selected from ethylenediamine and/or H12-MDA.

10. Process according to any of Claims 1 to 9, **characterized in that** the monohydroxy-functional alcohols are selected from aliphatic alcohols having one to six carbon atoms.

11. Process according to any of Claims 1 to 10, **characterized in that** at least one plasticizer and/or special-effect constituent is added to the nail varnish composition in the course of production.

12. Process according to any of Claims 1 to 11, **characterized in that** the nail polish composition is free of nitrocellulose.

13. Nail varnish composition obtainable by a process according to any of Claims 1 to 12.

14. Kit comprising a nail varnish composition according to Claim 13 and an alcohol-based nail varnish remover.

15. Process for producing a cosmetic coating on nails using nail varnish compositions according to Claim 13 or 14, wherein the cosmetic composition is applied to the nails.

## Patentansprüche

1. Verfahren zur Herstellung einer Nagellack-Zusammensetzung, **dadurch gekennzeichnet, dass** wenigstens ein Polyurethanharnstoff, welcher keine ionisch hydrophilierenden Gruppen aufweist und welcher in einem Lösemittel oder Lösemittelgemisch gelöst ist, eingesetzt wird, wobei das Lösemittel ausschließlich aus einem oder mehreren monohydroxyfunktionellen Alkoholen besteht oder ein Lösemittelgemisch ist, bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält,
wobei der Polyurethanharnstoff aufgebaut ist aus
a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung, die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einem zahlenmittleren Molekulargewicht Mn ≥ 500 und ≤ 6000 g/mol und einer Hydroxylfunktionalität von ≥ 1,5 und ≤ 4, welches unterschiedlich ist von b) .

2. Verfahren gemäß Anspruch 1, **dadurch gekennzeichnet, dass** der Polyurethanharnstoff keine hydrophilierenden Gruppen aufweist.

3. Verfahren gemäß Anspruch 1 oder 2, **dadurch gekennzeichnet, dass** die Komponente b) ausgewählt ist aus Po-ly(tetramethylenglykol)polyetherpolyolen.

4. Verfahren gemäß einem der Ansprüche 1 bis 3, **dadurch gekennzeichnet, dass** die Komponente b) ein zahlenmittleres Molekulargewicht Mn ≥ 500 und ≤ 2500 g/mol und eine Hydroxylfunktionalität von ≥ 1,9 und ≤ 3 aufweist.

5. Verfahren gemäß einem der Ansprüche 1 bis 4, **dadurch gekennzeichnet, dass** die Komponente a) ausgewählt ist aus aliphatischen, araliphatischen und/oder cycloaliphatischen Diisocyanaten, welche mindestens eine Isocyanatgruppe aufweisen, die an ein sekundäres und/oder tertiäres C-Atom gebunden ist.

6. Verfahren gemäß einem der Ansprüche 1 bis 5, **dadurch gekennzeichnet, dass** die Komponente a) ausgewählt ist aus IPDI und/oder H12-MDI.

7. Verfahren gemäß einem der Ansprüche 1 bis 6, **dadurch gekennzeichnet, dass** die Komponente c) ausgewählt ist aus Aminen, die mindestens zwei Aminogruppen aufweisen, die an primäre und/oder sekundäre C-Atome gebunden sind.

8. Verfahren gemäß einem der Ansprüche 1 bis 7, **dadurch gekennzeichnet, dass** die Komponente c) ausgewählt ist aus symmetrisch aufgebauten Diaminen.

9. Verfahren gemäß einem der Ansprüche 1 bis 8, **dadurch gekennzeichnet, dass** die Komponente c) ausgewählt ist aus Ethylendiamin und/oder H12-MDA.

10. Verfahren gemäß einem der Ansprüche 1 bis 9, **dadurch gekennzeichnet, dass** die monohydroxyfunktionellen Alkohole ausgewählt sind aus aliphatischen Alkohole mit einem bis sechs Kohlenstoffatomen.

11. Verfahren gemäß einem der Ansprüche 1 bis 10, **dadurch gekennzeichnet, dass** der Nagellack-Zusammensetzung bei der Herstellung wenigstens ein Weichmacher und/oder effektgebender Bestandteil zugesetzt wird.

12. Verfahren gemäß einem der Ansprüche 1 bis 11, **dadurch gekennzeichnet, dass** die Nagellack-Zusammensetzung frei von Nitrocellulose ist.

13. Nagellack-Zusammensetzung erhältlich nach einem Verfahren gemäß einem der Ansprüche 1 bis 12.

14. Kit umfassend eine Nagellack-Zusammensetzung nach Anspruch 13 sowie einen alkoholbasierten Nagellackentferner.

15. Verfahren zur Herstellung einer kosmetischen Beschichtung auf Nägeln unter Verwendung von Nagellack-Zusammensetzungen gemäß Anspruch 13 oder 14, wobei die kosmetische Zusammensetzung auf die Nägel aufgetragen wird.

## Revendications

1. Procédé de production d'une composition de vernis à ongles, **caractérisé en ce qu'**on utilise au moins un polyuréthane-urée ne possédant aucun groupement d'hydrophilisation par voie ionique et ayant été dissous dans un solvant ou un mélange de solvants, le solvant étant constitué de manière exclusive d'un ou plusieurs alcools à fonction monohydroxy ou étant un mélange de solvants constitué de solvants exclusivement organiques et contenant ≥ 50% en poids, sur la base de la masse totale du mélange de solvants, d'au moins un alcool à fonction monohydroxy,
où le polyuréthane-urée a été formé à partir
a) d'au moins un diisocyanate aliphatique, araliphatique et/ou cycloaliphatique,
b) d'au moins un polyéther polyol ayant un poids moléculaire moyen en nombre Mₙ ≥ 400 et ≤ 6000 g/mol et une fonctionnalité hydroxyle ≥ 1,5 et ≤ 4,
c) d'au moins un composé à fonction amino ayant au moins deux groupements amino réactifs avec l'isocyanate,
d) éventuellement d'au moins un alcool ayant au moins deux groupements hydroxyle et une masse molaire ≥ 60 et ≤ 399 g/mol,
e) éventuellement d'au moins un composé ayant un groupement réactif vis-à-vis des groupements isocyanate, et
f) éventuellement ≤ 20% en poids, sur la base de la masse totale du polyuréthane-urée, d'au moins un polyol différent de b) ayant un poids moléculaire moyen en nombre Mₙ ≥ 500 et ≤ 6000 g/mol et une fonctionnalité hydroxyle ≥ 1,5 et ≤ 4.

2. Procédé selon la revendication 1, **caractérisé en ce que** le polyuréthane-urée ne possède aucun groupement d'hydrophilisation.

3. Procédé selon la revendication 1 ou 2, **caractérisé en ce que** le composant b) est choisi parmi les poly(tétraméthylène glycol) polyéther polyols.

4. Procédé selon l'une quelconque des revendications 1 à 3, **caractérisé en ce que** le composant b) possède un poids moléculaire moyen en nombre Mₙ ≥ 500 et ≤ 2500 g/mol et une fonctionnalité hydroxyle ≥ 1,9 et ≤ 3.

5. Procédé selon l'une quelconque des revendications 1 à 4, **caractérisé en ce que** le composant a) est choisi parmi les diisocyanates aliphatiques, araliphatiques et/ou cycloaliphatiques ayant au moins un groupement isocyanate lié à un atome de carbone secondaire et/ou tertiaire.

6. Procédé selon l'une quelconque des revendications 1 à 5, **caractérisé en ce que** le composant a) est choisi parmi l'IPDI et/ou le H12-MDI.

7. Procédé selon l'une quelconque des revendications 1 à 6, **caractérisé en ce que** le composant c) est choisi parmi les amines ayant au moins deux groupements amino liés à des atomes de carbone primaires et/ou secondaires.

8. Procédé selon l'une quelconque des revendications 1 à 7, **caractérisé en ce que** le composant c) est choisi parmi les diamines symétriques.

9. Procédé selon l'une quelconque des revendications 1 à 8, **caractérisé en ce que** le composant c) est choisi parmi l'éthylènediamine et/ou la H12-MDA.

10. Procédé selon l'une quelconque des revendications 1 à 9, **caractérisé en ce que** les alcools à fonction monohydroxy sont choisis parmi les alcools aliphatiques ayant de un à six atomes de carbone.

11. Procédé selon l'une quelconque des revendications 1 à 10, **caractérisé en ce qu'**au moins un plastifiant et/ou constituant à effets spéciaux est ajouté à la composition de vernis à ongles au cours de la production.

12. Procédé selon l'une quelconque des revendications 1 à 11, **caractérisé en ce que** la composition de vernis à ongles est exempte de nitrocellulose.

13. Composition de vernis à ongles pouvant être obtenue par un procédé selon l'une quelconque des revendications 1 à 12.

14. Kit comprenant une composition de vernis à ongles selon la revendication 13 et un dissolvant pour vernis à ongles à base d'alcool.

15. Procédé de production d'un revêtement cosmétique sur les ongles à l'aide de compositions de vernis à ongles selon la revendication 13 ou 14, dans lequel la composition de vernis à ongles est appliquée sur les ongles.
